# EUROPEAN PATENT APPLICATION

(11) **EP 4 205 759 A1**
(43) Date of publication of application: **05.07.2023**
(21) Application number: 22150211.5
(22) Date of filing: 04.01.2022
(51) Int. Cl.: A61K 39/12

(54) **FOWL ADENOVIRUS SUBUNIT VACCINE AND PRODUCTION METHOD THEREOF**

(71) Applicant: Veterinärmedizinische Universität Wien, 1210 Vienna (AT)
(72) Inventor: HESS, Michael, 3400 Klosterneuburg (AT); SCHACHNER, Anna, 1200 Wien (AT)
(74) Representative: SONN Patentanwälte OG

(57) **Abstract**

The present invention provides a fowl adenovirus (FAdV) subunit vaccine, comprising at least a chimeric FAdV fiber protein and an adjuvant. This vaccine may be used to ameliorate or prevent adenoviral gizzard erosion (AGE), inclusion body hepatitis (IBH) or hepatitis-hydropericardium syndrome (HHS) in birds. The invention further relates to a method of producing an FAdV subunit vaccine, comprising the steps of expressing a chimeric FAdV fiber protein in an expression system, purifying the fiber protein, and combining the fiber protein with an adjuvant to obtain the FAdV subunit vaccine.

## Description

The field of the present invention relates to fowl adenovirus (FAdV) fiber subunit vaccines and methods of their production.

FAdVs, from the family Adenoviridae, genus Aviadenovirus, are non-enveloped double-stranded DNA viruses classified into five species (Fowl aviadenovirus A to Fowl aviadenovirus E, FAdV-A to FAdV-E) based on genomic features, and 12 subordinate serotypes (FAdV-1 to -8a, -8b to -11) defined by cross-neutralization (Harrach et al., 2012; Hess, 2020).

The FAdV-associated bird diseases hepatitis-hydropericardium syndrome (HHS) and inclusion body hepatitis (IBH) share characteristic pathogenic and immune mechanisms, being overall very distinct from those of adenoviral gizzard erosion (AGE). One of the underlying reasons appears to be a closer molecular relationship between the causative types of HHS (FAdV-4, species FAdV-C) and IBH (FAdV-2/-11, FAdV-D; FAdV-8a and -8b, FAdV-E) as compared to the genetically separated FAdV-1 (species FAdV-A) responsible for AGE (Schachner et al., 2018; Hess, 2020).

Both HHS and IBH are characterized by haemorrhages and dystrophic necrobiotic changes in the liver and kidneys, accompanied by intranuclear inclusion bodies. A characteristic pathological finding is the enlarged, dystrophic liver with yellowish colour and crumbly texture. More rarely, macroscopically visible necrotic foci could be detected in the liver. The kidneys are enlarged, pale and mottled with multiple haemorrhages. Sometimes, an atrophy of the bursa of Fabricius can be noticed. Often ecchymoses and striated haemorrhages in skeletal muscles are observed. Microscopically, extensive dystrophic changes and necroses of liver parenchyma are detected. In the nuclei of hepatocytes, basophilic or eosinophilic inclusion bodies are detected.

The diagnosis is based upon the typical gross lesions and the history records. In a number of cases, the dominant lesions are the prominent mottled or striated haemorrhages of the liver. In addition to those lesions HHS is characterized by a yellowish straw colored fluid in the hydropericard. Outbreaks are encountered primarily in meat type chickens, most commonly at the age of 1-8 weeks. In comparison to HHS various serotypes, predominantly seroytpes 2-11, are reported in the cause of IBH outbreaks.

Clinical signs can be observed already several hours prior to death occurrence. They consist in pale comb and wattles, depression and apathy. IBH is characterized by a sudden onset and a sharply increased death rate that reaches peak values by the 3rd - 4th day and returns back within the normal range by the 6th - 7th day. The total death rate is usually under 10% but can attain up to 30%, with up to 70% in case of HHS.

Despite worldwide distribution and severe economic impact of FAdV-associated diseases, commercially available immunization strategies rely mainly on the use of autogenous vaccines. Licenced products are mainly available to protect birds against HHS, developed after its first appearance. In Australia, a live vaccine based upon FAdV-8b was already developed in 1989 to control IBH (Steer et al. 2011). In recent years, the shortage of available vaccines has been tackled by experimental development of alternative vaccination antigens, including certain live and inactivated (Schonewille et al., 2010; Kim et al., 2014; Gupta et al., 2018; Popowich et al. 2018; Steer-Cope et al. 2019; Grafl et al., 2020), as well as subunit vaccines (Shah et al., 2012; Schachner et al., 2014; Wang et al. 2018; Schachner et al., 2018; Wang et al. 2019). In the majority of studies, structural proteins of the FAdV capsid were used for subunit vaccine formulation, based on their immunogenic potential and role in conferring antigenicity of the virus (Norrby, 1969). In particular, recombinant penton base and fiber, obtained from FAdV-4, proved successful for protecting chickens against the associated disease, HHS (Shah et al., 2012; Schachner et al., 2014; Chen et al., 2018; Wang et al., 2019). Finally, fiber-2 was integrated into recombinant Newcastle disease virus which could be used to protect birds from HHS albeit only after intramuscular vaccination (Tian et al. 2020).

WO 2015/024932 A1 discloses a subunit vaccine comprising fiber-2 protein of FAdV-C or an immunogenic fragment thereof. This vaccine can be used to prevent HHS in birds.

WO 2015/024929 A2 discloses a subunit vaccine comprising a fiber protein, selected from fiber-2 protein of FAdV-C, fiber-2 protein of FAdV-A, fiber protein of FAdV-B, FAdV-D and FAdV-E, or an immunogenic fragment thereof. This vaccine can be used to prevent HHS, IBH or gizzard erosion in birds.

However, vaccines with an extended protection spectrum are still needed due to simultaneous occurrence and mixed infections with diverse FAdV strains in the field (Gomis et al., 2006; Mittal et al., 2014; Schachner et al., 2016; Niu et al. 2018).

It is thus an object of the present invention to provide FAdV vaccines with an extended protection spectrum. In addition, these vaccines should be producible with reduced complexity and/or increased efficiency compared to FAdV vaccines in the prior art, in particular under good manufacturing practice (GMP) conditions.

The present invention provides an FAdV subunit vaccine (for birds, preferably poultry, especially chickens), comprising at least a chimeric FAdV fiber protein and an (immune-effective) adjuvant.

This vaccine is preferably for use in ameliorating or preventing AGE, IBH or HHS in birds, preferably in poultry, especially in chickens.

In another aspect, the present invention relates to a method of vaccinating a bird against an FAdV infection, comprising the steps of obtaining the inventive vaccine and administering the vaccine to the bird.

In yet another aspect, the present invention relates to a method of producing an FAdV subunit vaccine, comprising the steps of expressing a chimeric FAdV fiber protein in an expression system, purifying the fiber protein, and combining the fiber protein with an adjuvant (and preferably further vaccine components, such as pharmaceutically acceptable carriers and/or pharmaceutically acceptable diluents, in particular as disclosed herein) to obtain the FAdV subunit vaccine.

In the course of the present invention, it surprisingly turned out that FAdV subunit vaccines with chimeric fiber proteins are sufficiently immunogenic to offer an extended protection spectrum (e.g. when compared to an FAdV subunit vaccine based on a single FAdV strain). Using chimeric fiber proteins has the additional advantage of simplifying vaccine manufacturing compared to expressing and combining several different recombinant fiber proteins (e.g. from multiple serotypes) in a single vaccine, as chimeric fiber protein expression is easily possible in a single cell line (harboring the chimeric fiber gene) in a single bioreactor, the number of mixing steps during manufacturing can be reduced and 1:1 stoichiometry can be tightly controlled (the chimeric fiber is typically recombinantly expressed from a single gene). This advantage has particular significance when the FAdV vaccine is produced at industrial scale and/or under GMP conditions (which is required for veterinary medicinal products in most countries).

FAdV diseases, with discrete clinical pictures caused by particular serotypes, are an economic burden for the poultry industry on a worldwide scale. An increasing awareness of antigenically diverse FAdV types co-circulating in the field, and the demand for their control, explains why much research has been dedicated lately to elucidating broad-protectivity of candidate antigens, with less promising results so far. As a general paradigm, the immunity developed against a specific FAdV type does not confer protection against other types, a conclusion supported by observed shifts towards outbreaks with other viral types after implementation of a vaccination regimen against the previously dominating ones (Steer et *al.,* 2011; Venne, 2013; Wang et *al.,* 2018; Bertran et al 2021; Mo 2021).

The FAdV fiber is a surface antigen that features high levels of diversification between FAdV species and serotypes. Example 1 demonstrates cross-protection between different IBH-causing serotypes with the introduction of a novel chimeric fiber protein merging putative epitopes from both FAdV-8a and -8b fibers. In Example 2, a novel chimeric fiber retaining molecular characteristics of both FAdV-4 and -11 (termed "crecFib-4/11") was created. The new construct was then tested *in vivo* for its efficacy to immunize chickens against different FAdV-associated diseases, such as HHS and IBH, and performed well.

Unrelated to the present context, chimeric fiber proteins based on human adenovirus (HAdV) fiber proteins are disclosed in US 7,741,099. Such chimeric HAdV fiber proteins have an entirely different purpose, namely to circumvent pre-existing anti-adenoviral immunity in human hosts when using chimeric HAdV capsids as vectors e.g. for gene therapy. This purpose is of course opposite to the purpose of the inventive vaccine, namely to induce anti-adenoviral immunity in the host (i.e. bird). Accordingly, such chimeric HAdV fiber proteins in the prior art are neither used as a subunit vaccine nor together with an immune-effective adjuvant, let alone both.

According to a preferred embodiment, the chimeric FAdV fiber protein comprises an N-terminal fiber protein fragment from a first FAdV serotype and a C-terminal fiber protein fragment from a second (i.e. different) FAdV serotype.

For an even broader protection spectrum, it is preferred that the first FAdV serotype and the second FAdV serotype belong to different FAdV species (e.g. the first FAdV serotype belonging to FAdV-C, i.e. being FAdV-4, and the second FAdV serotype belonging to FAdV-D, or vice versa).

It is especially preferred that the first FAdV serotype belongs to the FAdV species FAdV-C and the second FAdV serotype belongs to an FAdV species selected from FAdV-B, FAdV-D and FAdV-E (e.g. FAdV-E), or vice versa.

In another embodiment, it is preferred that the first FAdV serotype belongs to the FAdV species FAdV-A and the second FAdV serotype belongs to an FAdV species selected from FAdV-B, FAdV-D and FAdV-E, or vice versa.

In yet another embodiment, it is preferred that the first FAdV serotype belongs to the FAdV species FAdV-C and the second FAdV serotype belongs to the FAdV species FAdV-A, or vice versa.

In another preferred embodiment, it is preferred that the first FAdV serotype belongs to an FAdV species selected from FAdV-B, FAdV-D and FAdV-E (in particular FAdV-E) and the second FAdV serotype belongs to an FAdV species selected from FAdV-B, FAdV-D and FAdV-E (in particular FAdV-E), or vice versa, preferably wherein two different species are selected.

According to another preferred embodiment, the first FAdV serotype is FAdV-4 and the second FAdV serotype is selected from FAdV-2, FAdV-11, FAdV-8a and FAdV-8b (in particular FAdV-11), or vice versa.

In another embodiment, it is preferred that the first FAdV serotype is FAdV-1 and the second FAdV serotype is selected from FAdV-2, FAdV-11, FAdV-8a and FAdV-8b, or vice versa.

In yet another embodiment, it is preferred that the first FAdV serotype is FAdV-4 and the second FAdV serotype is FAdV-1, or vice versa.

According to yet another preferred embodiment, the first FAdV serotype is selected from FAdV-2, FAdV-11, FAdV-8a and FAdV-8b (in particular FAdV-8b) and the second FAdV serotype is selected from FAdV-2, FAdV-11, FAdV-8a and FAdV-8b (in particular FAdV-8a), or vice versa - preferably under the proviso that two different FAdV serotypes are selected.

The vaccine of the present invention is typically cross-protective, i.e. induces (at least partial) immune protection against at least two FAdV serotypes (from the same or different FAdV species) in a bird, preferably in poultry, more preferably in a chicken.

It is highly preferred that both the N-terminal fiber protein fragment and the C-terminal fiber protein fragment of the chimeric FAdV fiber protein are immunogenic in a bird such as a chicken.

To this end, it is highly preferred that the N-terminal fiber protein fragment comprises at least one, preferably at least two, especially at least three (B-cell) epitopes and the C-terminal fiber protein fragment comprises at least one, preferably at least two, especially at least three (B-cell) epitopes. Such epitopes may be confirmed experimentally or predicted with methods known in the art, e.g. with the DiscoTope 2.0 software (Vindahl Kringelum et al, 2012).

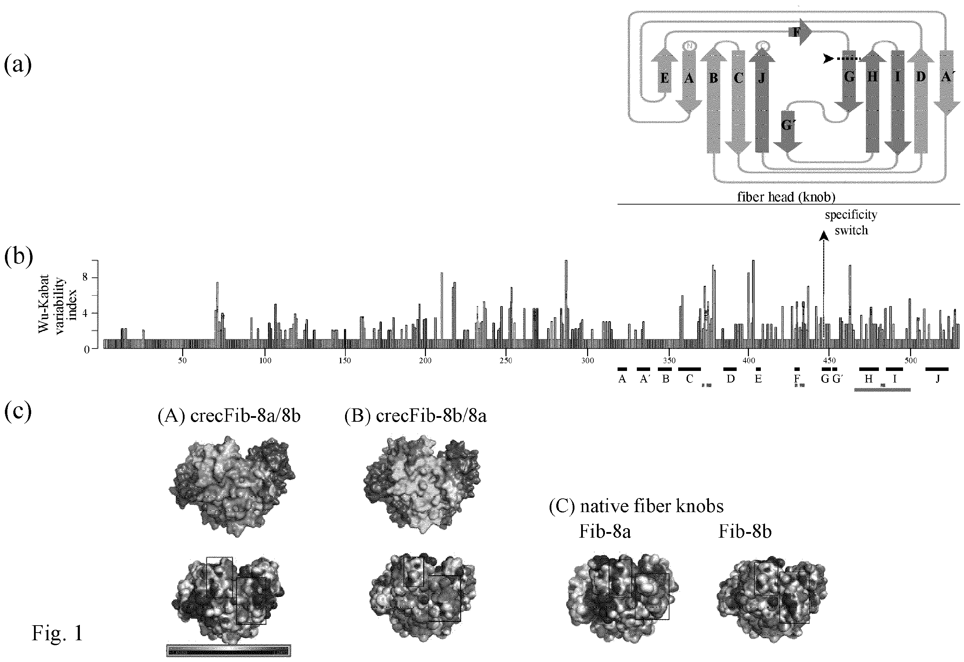

In the course of the present invention, it turned out that chimeric FAdV fiber proteins having the "specificity switch" within the fiber knob domain (see Examples 1 and 2, and in particular Fig. 1) were particularly suitable for the purposes of the present invention. Thus, in another preferred embodiment, the N-terminal fiber protein fragment comprises a fiber shaft domain and an N-terminal part of a fiber knob domain, wherein the C-terminal fiber protein fragment comprises a C-terminal part of a fiber knob domain.

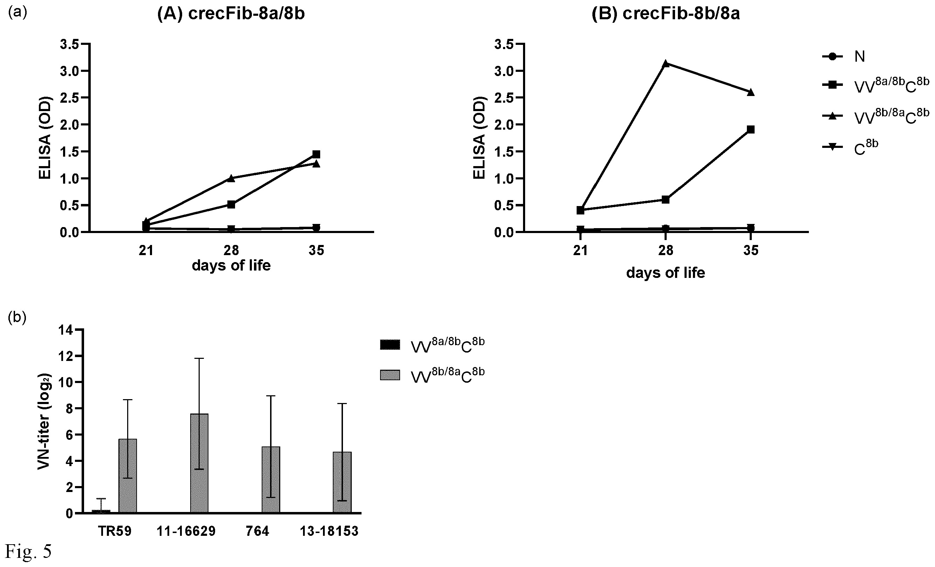

For the present invention, a fragment of fiber protein (in particular fiber-2) of FAdV-C or FAdV-A or of fiber protein of FAdV-B, FAdV-D or FAdV-E may be used as fiber protein fragment (e.g. N-terminal or C-terminal fiber protein fragment) of the chimeric FAdV fiber protein. Many FAdV fiber protein sequences are known to the skilled person, e.g. from reference strains KR5 (HE608152) or CFA20 (AF160185) or strain ON1 (GU188428 = NC_015323) or any other FAdV-C field isolates, e.g. isolates IV37, K99-97, K388-95, K88-95, K31, Peru53, Peru54, c344, K1013, AG234, C2B, 09-584, 09-8846, 09-2602, 922-1, Da60, K1013QT and INT4 (as disclosed by Marek et al., 2012)); and CELO (FAdV-A; Q64787) 340 (FAdV-B), A2-A (FAdV-D; AC000013), HG (FAdV-E; GU734104); corresponding to UniProt entries H8WG65, H8WG69, H8WG72, H8WG77, H8WG70, H8WG73, H8WG66, H8WG76, H8WG60, H8WG61, H8WG62, H8WG75, H8WG67, H8WG78, H8WG63, H8WG68, H8WG64, H8WG74, H8WG71, H8WQZ7, H8WQZ2, H8WQW9, QOGH78, 055281, and F2VJI5. Further examples of FAdV fiber proteins are for instance listed in Table 3 of WO 2015/024929 A2 and Fig. 5a-5i of WO 2015/024929 A2.

In particular, any of the FAdV fiber fragments disclosed in Examples 1 and 2 may be used for the present invention (more specifically, the amino acid sequence translated therefrom), preferably in the combination and order disclosed in these examples.

Accordingly, it is preferred that the N-terminal fiber protein fragment comprises an amino-acid sequence at least 70%, preferably at least 80%, more preferably at least 90%, even more preferably at least 95% or even 97.5%, especially 99% or even completely identical to any one of the following sequences:
FAdV-8a fiber N-terminal fragment (SEQ ID NO: 1): 
MATSTPHAFSFGQIGSRKRPAGGDGERDASKVPKMQTPAPSATANGNDELDLVYPFWLQNGSTGGGGGGGSGGNPSL
NPPFLDPNGPLTVQNNLLKVNTTAPIAVTNKALTLSFDAESLELTNQQQLAVKIDPEGPLKATTEGIQLSVDPTTLE
VDDVDWELTVNLDPNGPLDSSATGITVRVDDTLLIDDDGSGQGKELGVHLNPDGPITADQNGLDLEIDNQTLKITPG
SAGGVLSVQLKPQGGLNSQSDGIQVVTQNSIEVDNGALDVKVVANGPLSTTPNGLTLNYDTGDFTVNAGTLSILRNP
SLVANAYLTSGARTLQQFTAKGENSSQFSFPCAYYLQQWLSDGLIFSSLYLKLDRTRFTGMSSDPSYQNARYFTFWV
GGGAAMNLSQLSTPTITPSTTEWTAFAPALNYSGAPAFVYDANPVFTIYFQPNTGR
FAdV-8b fiber N-terminal fragment (SEQ ID NO: 2): 
MATSTPHAFSFGQIGSRKRPAGGDGERDASKVPKMQTPAPSATANGNDELDLVYPFWLQNGSTGGGGGGGSGGNPSL
NPPFLDPNGPLTVQNNLLKVNTTAPIAVTNKALTLAYESDSLELTNQQQLAVKIDPEGPLKATTEGIQLSVDPTTLE
VDDVDWELTVKLDPDGPLDSSATGITVRVDETLLIEDDGSGQGKELGVNLNPTGPITADDQGLDLEIDNQTLKVNSV
TGGGVLAVQLKSQGGLTAQTDGIQVNTQNSITVTNGALDVKVAANGPLESTDTGLTLNYDPGDFTVNAGTLSIIRDP
ALVANAYLTSGASTLQQFTAKSENSSQFSFPCAYYLQQWLSDGLIFSSLYLKLDRAQFTNMPTGANYQNARYFTFWV
GAGTSFNLSTLTEPTITPNTTQWNAFAPALDYSGAPPFIYDASSVVTIYFEPTSGR
FAdV-4 fiber-2 N-terminal fragment (SEQ ID NO: 3): 
MLRAPKRRHSENGQPESEAGPSPAPIKRAKRMVRASQLDLVYPFDYVADPVGGLNPPFLGGSGPLVDQGGQLTLNVT
DPIIIKNRSVDLAHDPSLDVNAQGQLAVAVDPEGALDITPDGLDVKVDGVTVMVNDDWELAVKVDPSGGLDSTAGGL
GVSVDDTLLVDQGELGVHLNQQGPITADSSGIDLEINPNMFTVNTSTGSGVLELNLKAQGGIQAGSSGVGVSVDESL
EIVNNTLEVKPDPSGPLTVSANGLGLKYDSNTLAVTAGALTVVGGGSVSTPIATFVSGSPSLNTYNATIVNSSSHPF
SCAYYLQQWNVQGLLFTSLYVKLDSTTMGTRPGDNSSANAKWFTFWVSAYLQQCNPSGIQAGTVSPSTAALADFEPM
ANRSVSSPWTYSANAYYQPSSG
FAdV-11 fiber N-terminal fragment (SEQ ID NO: 4):

MAKSTPFTFSMGQHSSRKRPADSENTQNASKVAKTQTSATRAGVDGNDDLNLVYPFWLQNSTSGGGGGGSGGNPSLN
PPFIDPNGPLYVQNSLLYVKTTAPIEVENKSLALAYDSSLDVDAQNQLQVKVDAEGPIRISPDGLDIAVDPSTLEVD
DEWELTVKLDPAGPISSSSAGINIRVDDTLLIEDDDTAQVKELGVHLNPNGPITADQDGLDLEVDPQTLTVTTSGAT
GGVLGVLLKPSGGLQTNIQGIGVAVADTLTISSNTVEVKTDPNGSIGSSSNGIAVVTDPAGPLTTSSNGLSLKLTPN
GSIQSSSTGLSVQTDPAGPITSGANGLSLSYDTSDFTVSQGMLSIIRNPSTYPDAYLESGTNLLNNYTAYAENSSNY
KFNCAYFLQSWYSNGLVTSSLYLKINRDNLNSLPSGQLSENAKYFTFWVPTYESMNLSNVATPTITPSSVPWGAFLP
AQNCTSNPAFKYYLTQPPSIYFEPESG

Alternatively, or in addition thereto, it is preferred that the C-terminal fiber protein fragment comprises an amino-acid sequence at least 70%, preferably at least 80%, more preferably at least 90%, even more preferably at least 95% or even 97.5%, especially 99% or even completely identical to any one of the following sequences:
FAdV-8a fiber C-terminal fragment (SEQ ID NO: 5): 
VDTYLPVLTGDWKTSSTYNPGTITLVVRNATIQLQSQSTFTTSVCYNFRCQNSGIFNNNATSGTLTLGPIFYSCPAL
STADVS
FAdV-8b fiber C-terminal fragment (SEQ ID NO: 6): 
LESYLPVLTDNWSQTYNPGTVTLCVKTVRVQLRSQGTFSTLVCYNFRCQNTGIFNSNATAGTMTLGPIFFSCPALST
ANAP
FAdV-11 fiber C-terminal fragment (SEQ ID NO: 7): 
SVQTFQPVLTGDWDTNTYNPGTVQVCILPQTVVGGQSTFVNMTCYNFRCQNPGIFKVAASSGTFTIGPIFYSCPTNK
LTQP
FAdV-4 fiber-2 C-terminal fragment (SEQ ID NO: 8):
   EFQVFTPVVTGAWNPGNIGIRVLPVPVTASGDRYTLLCYSLQCTNSSIFNPANSGTMIVGPVLYSCPAASVP

In a particularly preferred embodiment, the chimeric FAdV fiber protein comprises an amino-acid sequence at least 70%, preferably at least 80%, more preferably at least 90%, even more preferably at least 95% or even 97.5%, especially 99% or even completely identical to any one of the following sequences:
FAdV-8a/8b chimera (SEQ ID NO: 9): 
MATSTPHAFSFGQIGSRKRPAGGDGERDASKVPKMQTPAPSATANGNDELDLVYPFWLQNGSTGGGGGGGSGGNPSL
NPPFLDPNGPLTVQNNLLKVNTTAPIAVTNKALTLSFDAESLELTNQQQLAVKIDPEGPLKATTEGIQLSVDPTTLE
VDDVDWELTVNLDPNGPLDSSATGITVRVDDTLLIDDDGSGQGKELGVHLNPDGPITADQNGLDLEIDNQTLKITPG
SAGGVLSVQLKPQGGLNSQSDGIQVVTQNSIEVDNGALDVKVVANGPLSTTPNGLTLNYDTGDFTVNAGTLSILRNP
SLVANAYLTSGARTLQQFTAKGENSSQFSFPCAYYLQQWLSDGLIFSSLYLKLDRTRFTGMSSDPSYQNARYFTFWV
GGGAAMNLSQLSTPTITPSTTEWTAFAPALNYSGAPAFVYDANPVFTIYFQPNTGRLESYLPVLTDNWSQTYNPGTV
TLCVKTVRVQLRSQGTFSTLVCYNFRCQNTGIFNSNATAGTMTLGPIFFSCPALSTANAP
FAdV-8b/8a chimera (SEQ ID NO: 10): 
MATSTPHAFSFGQIGSRKRPAGGDGERDASKVPKMQTPAPSATANGNDELDLVYPFWLQNGSTGGGGGGGSGGNPSL
NPPFLDPNGPLTVQNNLLKVNTTAPIAVTNKALTLAYESDSLELTNQQQLAVKIDPEGPLKATTEGIQLSVDPTTLE
VDDVDWELTVKLDPDGPLDSSATGITVRVDETLLIEDDGSGQGKELGVNLNPTGPITADDQGLDLEIDNQTLKVNSV
TGGGVLAVQLKSQGGLTAQTDGIQVNTQNSITVTNGALDVKVAANGPLESTDTGLTLNYDPGDFTVNAGTLSIIRDP
ALVANAYLTSGASTLQQFTAKSENSSQFSFPCAYYLQQWLSDGLIFSSLYLKLDRAQFTNMPTGANYQNARYFTFWV
GAGTSFNLSTLTEPTITPNTTQWNAFAPALDYSGAPPFIYDASSVVTIYFEPTSGRVDTYLPVLTGDWKTSSTYNPG
TITLVVRNATIQLQSQSTFTTSVCYNFRCQNSGIFNNNATSGTLTLGPIFYSCPALSTADVS
FAdV-4/11 chimera (SEQ ID NO: 11): 
MLRAPKRRHSENGQPESEAGPSPAPIKRAKRMVRASQLDLVYPFDYVADPVGGLNPPFLGGSGPLVDQGGQLTLNVT
DPIIIKNRSVDLAHDPSLDVNAQGQLAVAVDPEGALDITPDGLDVKVDGVTVMVNDDWELAVKVDPSGGLDSTAGGL
GVSVDDTLLVDQGELGVHLNQQGPITADSSGIDLEINPNMFTVNTSTGSGVLELNLKAQGGIQAGSSGVGVSVDESL
EIVNNTLEVKPDPSGPLTVSANGLGLKYDSNTLAVTAGALTVVGGGSVSTPIATFVSGSPSLNTYNATIVNSSSHPF
SCAYYLQQWNVQGLLFTSLYVKLDSTTMGTRPGDNSSANAKWFTFWVSAYLQQCNPSGIQAGTVSPSTAALADFEPM
ANRSVSSPWTYSANAYYQPSSGSVQTFQPVLTGDWDTNTYNPGTVQVCILPQTVVGGQSTFVNMTCYNFRCQNPGIF
KVAASSGTFTIGPIFYSCPTNKLTQP
FAdV-11/4 chimera (SEQ ID NO: 12):

MAKSTPFTFSMGQHSSRKRPADSENTQNASKVAKTQTSATRAGVDGNDDLNLVYPFWLQNSTSGGGGGGSGGNPSLN
PPFIDPNGPLYVQNSLLYVKTTAPIEVENKSLALAYDSSLDVDAQNQLQVKVDAEGPIRISPDGLDIAVDPSTLEVD
DEWELTVKLDPAGPISSSSAGINIRVDDTLLIEDDDTAQVKELGVHLNPNGPITADQDGLDLEVDPQTLTVTTSGAT
GGVLGVLLKPSGGLQTNIQGIGVAVADTLTISSNTVEVKTDPNGSIGSSSNGIAVVTDPAGPLTTSSNGLSLKLTPN
GSIQSSSTGLSVQTDPAGPITSGANGLSLSYDTSDFTVSQGMLSIIRNPSTYPDAYLESGTNLLNNYTAYAENSSNY
KFNCAYFLQSWYSNGLVTSSLYLKINRDNLNSLPSGQLSENAKYFTFWVPTYESMNLSNVATPTITPSSVPWGAFLP
AQNCTSNPAFKYYLTQPPSIYFEPESGEFQVFTPVVTGAWNPGNIGIRVLPVPVTASGDRYTLLCYSLQCTNSSIFN
PANSGTMIVGPVLYSCPAASVP

The vaccine according to the present invention comprises an (immuno-effective amount of an) adjuvant, preferably selected from the group consisting of Freund's complete adjuvant, Freund's incomplete adjuvant, aluminum hydroxide, Bordetella pertussis, saponin, muramyl dipeptide, ethylene vinyl acetate copolymer, oil, a vegetable oil or a mineral oil, in particular peanut oil or silicone oil, and combinations thereof.

Adjuvants are substances that enhance the immune response to immunogens (specifically, the chimeric FAdV fiber protein). Adjuvants can include aluminum hydroxide and aluminum phosphate, saponins e.g., Quil A, water-in-oil emulsion, oil-in-water emulsion, water-in-oil-in-water emulsion. The emulsion can be based in particular on light liquid paraffin oil (European Pharmacopea type); isoprenoid oil such as squalane or squalene; oil resulting from the oligomerization of alkenes, in particular of isobutene or decene; esters of acids or of alcohols containing a linear alkyl group, more particularly plant oils, ethyl oleate, propylene glycol di(caprylate/caprate), glyceryl tri(caprylate/caprate) or propylene glycol dioleate; esters of branched fatty acids or alcohols, in particular isostearic acid esters. The oil is used in combination with emulsifiers to form the emulsion. The emulsifiers are preferably nonionic surfactants, in particular esters of sorbitan, of mannide (e.g. anhydromannitol oleate), of glycerol, of polyglycerol, of propylene glycol and of oleic, isostearic, ricinoleic or hydroxystearic acid, which are optionally ethoxylated, and polyoxypropylene-polyoxyethylene copolymer blocks, in particular the Pluronic(R) products, especially L121. For example, the adjuvant-containing vaccine is prepared in the following way: 50 to 90 v/v of aqueous phase comprising the immunogen are emulsified in 1 to 10% w/v of anhydromannitol oleate, 1 to 10% w/v of oleic acid ethoxylated with 11 EO (ethylene oxide) and 5 to 40% v/v of light liquid paraffin oil (European Pharmacopea type) with the aid of an emulsifying turbomixer. An alternative method for preparing the emulsion consists in emulsifying, by passages through a highpressure homogenizer, a mixture of 1 to 10% w/v squalane, 1 to 10% w/v Pluronic(R) L121, 0.05 to 1% w/v of an ester of oleic acid and of anhydrosorbitol ethoxylated with 20 EO, 50 to 95% v/v of the aqueous phase comprising the immunogen. It is also possible to formulate with synthetic polymers (e.g., homo- and copolymers of lactic and glycolic acid, which have been used to produce microspheres that encapsulate immunogens, e.g., biodegradable microspheres) . A further instance of an adjuvant is a compound chosen from the polymers of acrylic or methacrylic acid and the copolymers of maleic anhydride and alkenyl derivative. Advantageous adjuvant compounds are the polymers of acrylic or methacrylic acid which are cross-linked, especially with polyalkenyl ethers of sugars or polyalcohols. These compounds are known by the term carbomer, e.g. acrylic polymers cross-linked with a polyhydroxylated compound having at least 3 hydroxyl groups, preferably not more than 8, the hydrogen atoms of at least three hydroxyls being replaced by unsaturated aliphatic radicals having at least 2 carbon atoms. The preferred radicals are those containing from 2 to 4 carbon atoms, e.g. vinyls, allyls and other ethylenically unsaturated groups. The unsaturated radicals may themselves contain other substituents, such as methyl. The products sold under the name Carbopol(R) (BF Goodrich, Ohio, USA) are particularly appropriate. They are cross-linked with an allyl sucrose or with allyl pentaerythritol. Among then, there may be mentioned Carbopol(R) 974P, 934P and 971P. Among the copolymers of maleic anhydride and alkenyl derivative, the copolymers EMA(R) (Monsanto) which are copolymers of maleic anhydride and ethylene, linear or cross-linked, for example cross-linked with divinyl ether, are preferred. The dissolution of these polymers in water leads to an acid solution that will be neutralized, preferably to physiological pH, in order to give the adjuvant solution into which the immunogenic, immunological or vaccine composition itself will be incorporated. The carboxyl groups of the polymer are then partly in COO- form.

In another preferment, a solution of adjuvant is prepared in distilled water, preferably in the presence of sodium chloride, the solution obtained being at acidic pH. This stock solution is diluted by adding it to the desired quantity (for obtaining the desired final concentration), or a substantial part thereof, of water charged with NaCl, preferably physiological saline (NaCl 9 g/1) all at once in several portions with concomitant or subsequent neutralization (pH 7.3 to 7.4), preferably with NaOH. This solution at physiological pH will be used as it is for mixing with the immunogen, which may be especially stored in freeze-dried, liquid or frozen form.

The vaccine according to the present invention preferably comprises a pharmaceutically acceptable diluent and/or carrier, preferably selected from the group consisting of water-forinjection, physiological saline, tissue culture medium, propylene glycol, polyethylene glycol, vegetable oils, especially olive oil, and injectable organic esters such as ethyl oleate.

The inventive vaccine is preferably for use in ameliorating or preventing AGE, IBH or HHS in birds, preferably in poultry, especially in chickens.

In other words, the inventive vaccine is preferably for use in immunizing birds, preferably poultry, especially chickens against an FAdV infection (in particular an infection with the first and/or the second FAdV serotype).

For the inventive prophylactic use or method, the vaccine is administered to the birds in an effective amount at a suitable point in time. Typical ways of administration are intravenous, subcutaneous, intramuscular, oral, in-ovo, intranasal, eye drop or intracloacal administration. Preferably, vaccination in chicken is effected up to week 19.

According to another preferred embodiment, the vaccine is administered to the birds at least twice. It turned out that a booster is particularly helpful to increase antibodies against the chimeric fiber protein.

The chimeric FAdV fiber protein can be produced by any suitable expression system. Preferably, production is effected in a eukaryotic expression system. Specifically preferred expression systems are a baculovirus expression system, bacterial expression systems such as an *E. coli* expression system, or a *Pichia pastoris* expression system. However, virtually any suitable expression system or vector can be used in the production of the vaccine provided by this invention. By way of illustration, said suitable expression or vector systems can be selected, according to the conditions and needs of each specific case, from plasmids, bacmids, yeast artificial chromosomes (YACs), bacteria artificial chromosomes (BACs), bacteriophage P1-based artificial chromosomes (PACs), cosmids, or viruses, which can further have a heterologous replication origin, for example, bacterial or of yeast, so that it may be amplified in bacteria or yeasts, as well as a marker usable for selecting the transfected cells different from the gene or genes of interest. These expression systems or vectors can be obtained by conventional methods known by persons skilled in the art.

Upon expression, the chimeric FAdV fiber protein may then be purified by any suitable protein purification method known in the art, e.g. by affinity chromatography, in case the chimeric FAdV fiber protein has a His tag or another affinity tag. Such affinity tag may be cleaved before formulating the final dosage form of the vaccine.

The vaccine according to the present invention can be produced in industrial amounts; the individual vaccine dose given to the animals can be in the ranges also applied for other vaccines. Preferably, the chimeric FAdV fiber protein is contained in the vaccine in an amount of 0.1 µg/ml to 10 mg/ml, preferably of 1 µg/ml to 1 mg/ml, especially of 10 to 100 µg/ml.

The vaccine according to the present invention preferably comprises a pharmaceutically acceptable diluent, especially if provided as commercially sold vaccine product. The suitable vehicles may be both aqueous and non-aqueous. Examples of non-aqueous solvents are propylene glycol, polyethylene glycol, vegetable oils such as olive oil, and injectable organic esters such as ethyl oleate.

According to a preferred embodiment, the vaccine according to the present invention is formulated as a dose form, i.e. it is already formulated to be administered without further partition/formulation/separation steps.

The vaccine of the present invention typically does not contain nucleic acids and/or typically does not contain any viral vectors (i.e. is vector-free) or viral capsids.

Herein, "UniProt" refers to the Universal Protein Resource. UniProt is a comprehensive resource for protein sequence and annotation data. UniProt is a collaboration between the European Bioinformatics Institute (EMBL-EBI), the SIB Swiss Institute of Bioinformatics and the Protein Information Resource (PIR). Across the three institutes more than 100 people are involved through different tasks such as database curation, software development and support. Website: http://www.uniprot.org/

Entries in the UniProt databases are identified by their accession codes (referred to herein e.g. as "UniProt accession code" or briefly as "UniProt" followed by the accession code), usually a code of six alphanumeric letters (e.g. "H8WG77"). If not specified otherwise, the accession codes used herein refer to entries in the Protein Knowledgebase (UniProtKB) of UniProt. If not stated otherwise, the UniProt database state for all entries referenced herein (or in WO 2015/024929 A2) shall be of 10 February 2021 (UniProt/UniProtKB Release 2021_01).

In the context of the present application, sequence variants (designated as "natural variant" in UniProt) are expressly included when referring to a UniProt database entry.

"Percent (%) amino acid sequence identity" or "X% identical" (such as "70% identical") with respect to a reference polypeptide or protein sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, ALIGN-2, Megalign (DNASTAR) or the "needle" pairwise sequence alignment application of the EMBOSS software package. Those skilled in the art can determine appropriate parameters for aligning sequences, including any algorithms needed to achieve maximal alignment over the full length of the sequences being compared. For purposes herein, however, % amino acid sequence identity values are calculated using the sequence alignment of the computer programme "needle" of the EMBOSS software package (publicly available from European Molecular Biology Laboratory; Rice et al., 2000).

The needle programme can be accessed under the web site http://www.ebi.ac.uk/Tools/psa/emboss_needle/ or downloaded for local installation as part of the EMBOSS package from http://emboss.sourceforge.net/. It runs on many widely-used UNIX operating systems, such as Linux.

To align two protein sequences, the needle programme is preferably run with the following parameters:

```
      Commandline: needle -auto -stdout -asequence
 SEQUENCE_FILE_A -bsequence SEQUENCE_FILE_B -datafile EBLOSUM62 -
 gapopen 10.0 -gapextend 0.5 -endopen 10.0 -endextend 0.5 -
 aformat3 pair -sprotein1 -sprotein2 (Align_format: pair
 Report_file: stdout)
```

The % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y where X is the number of amino acid residues scored as identical matches by the sequence alignment program needle in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. In cases where "the sequence of A is more than N% identical to the entire sequence of B", Y is the entire sequence length of B (i.e. the entire number of amino acid residues in B). Unless specifically stated otherwise, all % amino acid sequence identity values used herein are obtained as described in the immediately preceding paragraph using the needle computer program.

The present invention is further illustrated by the following figures and examples, without being restricted thereto.
**Figure 1****.** (a) The crecFib knob domain, represented schematically as a 2-dimensional ribbon model (adapted from El Bakkouri et al., 2008). Arrows represent β-strands (designated A to J, in linear order from the N- to the C-terminus of the knob sequence) connected by lines representing the intervening loops. Structures encoded by sequences of discriminate serotypes are shown in different colours opposite of the chimeric exchange site (the "specificity switch", indicated by the dashed line at the left end of the G strand). (b) Coordinates of β-strands of the fiber knob (black bars), candidate epitopes (blue bars, epitopes predicted by DiscoTope 2.0; red bar, a previously reported epitope by Wang et al., 2018) and the specificity switch, annotated in a Wu-Kabat plot of the FAdV-E fiber sequence. The degree of variability, indicated by the bar size for each residue position, has been calculated as the Wu-Kabat index from available fibers representing all types of the species (N=29). Amino acid positions of the multiple sequence alignment are annotated along the horizontal axis. Candidate epitopes conform well with less tightly conserved residues, while the specificity switch is located at an intraspecies consensus site. (c) Comparisons of the two crecFib knobs (8a/8b and 8b/8a) at similarity modeling of the amino acid sequence (A and B) and their predicted surface electrostatics in the panels underneath, with the molecule shown in side view. In the top panel only one chain of the fiber trimer is coloured with orange tones for the sequence portion derived from FAdV-8a, and green tones for the sequence portion from FAdV-8b; muted tones indicate the three epitopes predicted in this study. The same epitopes are highlighted with black squares in the electrostatic surface charge images with colour transitions from red (negative charge) to blue (positive charge), and are compared to the corresponding sites in the cognate native knobs of FAdV-8a and - 8b (C).

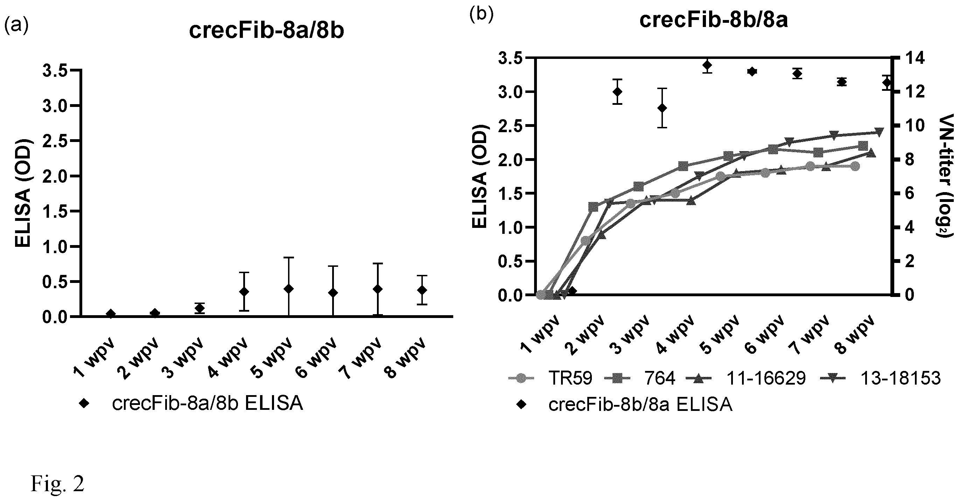

**Figure 2****.** Antibody development following immunization with crecFib constructs. The header of each panel indicates the construct used for immunization, which also corresponds to the coating antigen of the ELISA for determination of weekly ODs from 1-8 weeks post vaccination (wpv). Mean neutralization titers, if detectable, are plotted with the respective colours for the viruses against which the sera were tested (including the chimeric template, and the challenge strains of this study from FAdV-8a and -8b) .

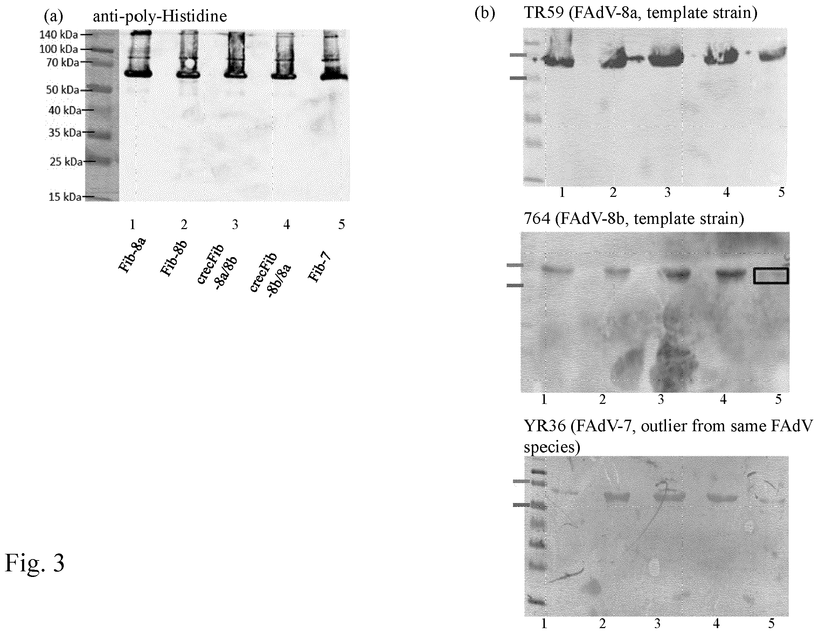

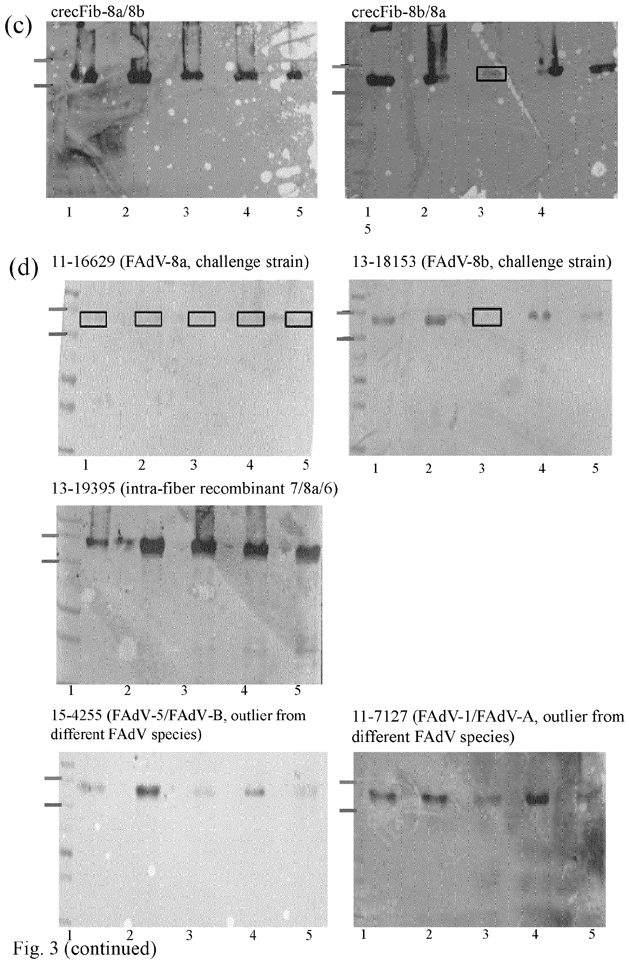

**Figure 3****.** Western blot analysis to assess the reactivity of crecFib constructs side-by-side with monospecific control fibers from types FAdV-8a, FAdV-8b and FAdV-7 (all species FAdV-E). The order of antigens is the same on all membranes, as labelled for the first membrane: 1, Fib-8a; 2, Fib-8b; 3, crecFib-8a/8b; 4, crecFib-8b/8a; 5, Fib-7. However, each membrane was incubated with a different antiserum specified in the header of each membrane, with the corresponding serotype in parentheses. Molecular weight sizes are indicated by the standard in the first lane in (a); in all subsequent blots only the relevant 50 kDa to 70 kDa range is marked. Signals which are present, but difficult to distinguish from the background, are boxed. (a) Confirmation of monomeric fibers via the poly-Histidine tag. (b) Detection of crecFib by antisera raised against the constitutive strains from FAdV-8a and -8b, and FAdV-7 as outlier type. (c) Detection of crecFib by antisera from birds vaccinated with the same, as well as the reverse-order construct. CrecFib antisera also recognize all monospecific control fibers, (d) Detection of crecFib and control fibers by antisera against field isolates, including the two challenge strains of this study, a naturally recombinant strain and exemplary strains from outside species FAdV-E with either one (FAdV-B) or two fiber genes (FAdV-A).

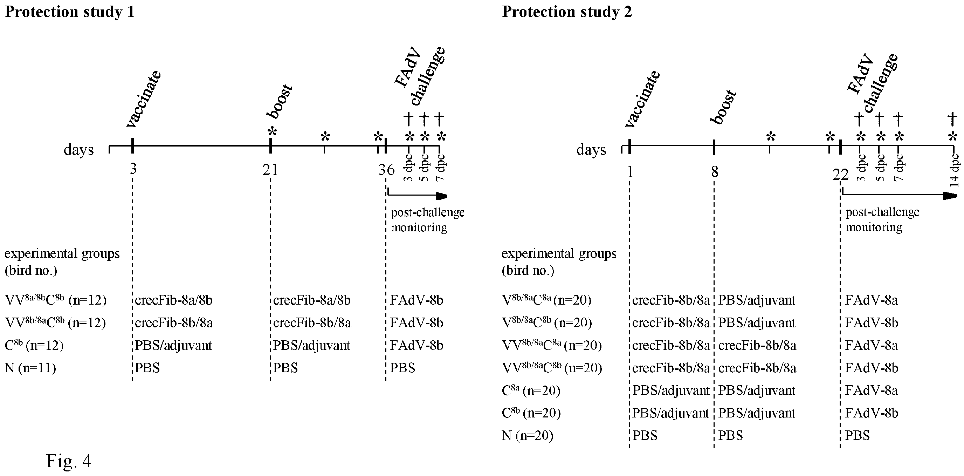

**Figure 4****.** Experimental design of Protection studies 1 and 2. The procedures (vaccination, booster, challenge) carried out on birds are indicated on the timelines (as days of life and days post challenge = dpc). Individual treatment schemes of each group are specified below. Asterisks indicate blood sampling, cross symbols indicate sequential killings, with necropsy and organ sampling.
**Figure 5****.** Prior-challenge antibody development in Protection study 1 (prime-boost vaccination with crecFib-8a/8b or crecFib-8b/8a). (a) Mean OD values for each treatment (groups receiving the same treatment were pooled), measured by crecFib-8a/8b ELISA (A) and crecFib-8b/8a ELISA (B) at 21 (18 dpv), 28 (7 dpb) and 35 (14 dpb) days of life. (b) Neutralizing antibody titers at 35 days of life (14 dpb = immediately prior challenge) against FAdV-8a (strains TR59 and 11-16629) and FAdV-8b (strains 764 and 13-18153) .

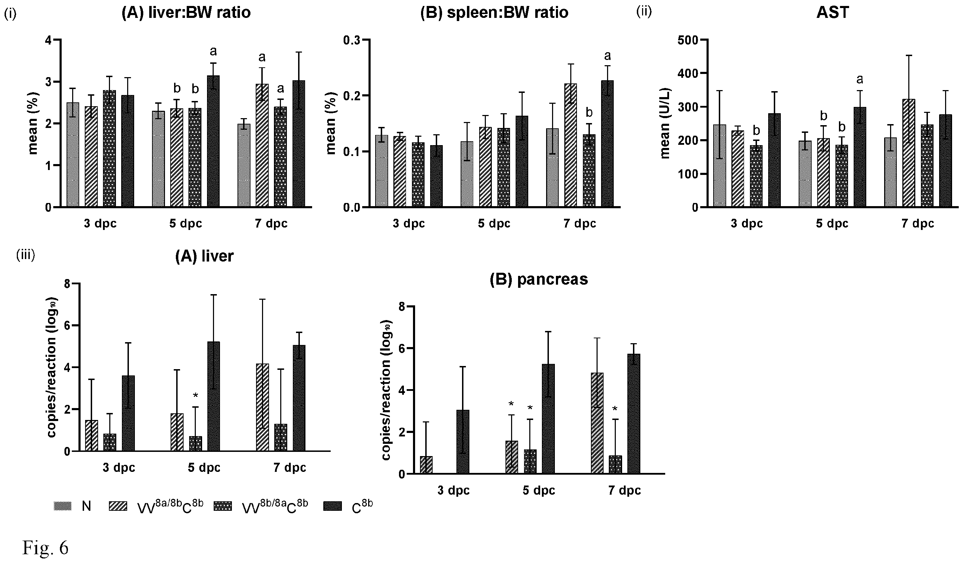

**Figure 6****.** Endpoints of protection, evaluated after FAdV-8b challenge in Protection study 1 (at 3, 5 and 7 dpc). (i) Organbody weight (BW) ratios for liver (A) and spleen (B). Lowercase letters above bars indicate significant differences (vs. negative control, "a"; vs. challenge control, "b"). (ii) Plasma AST (significantly different vs. negative control, "a"; significantly different vs. challenge control, "b"). (iii) Viral load in liver (A) and pancreas (B). Significant differences vs. challenge control are indicated with asterisks.

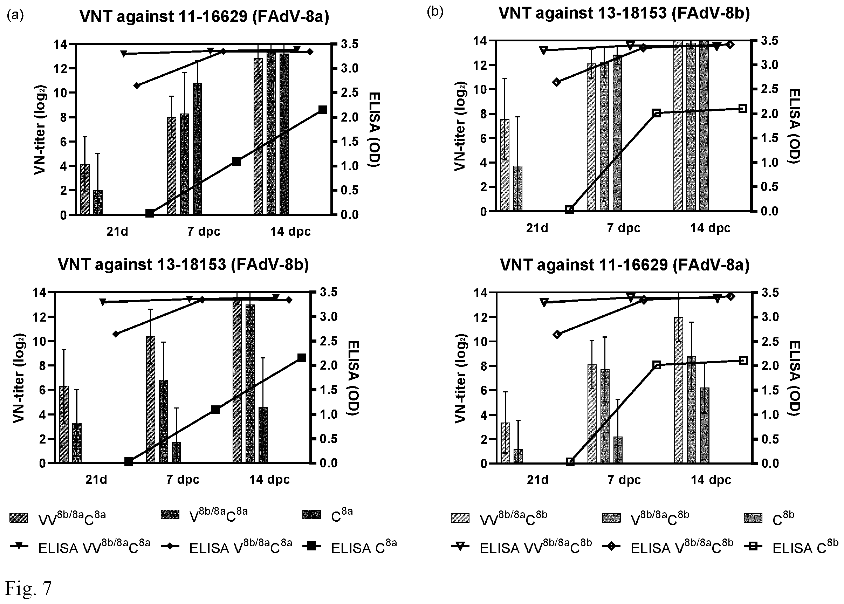

**Figure 7****.** Antibody development (ELISA and VNT) in Protection study 2 (comparing the V^{8b/8a} and VV^{8b/8a} regimens against challenge with either FAdV-8a or -8b) immediately prior (21 d), and after challenge (7 and 14 dpc). Only groups that received the same challenge were compared among each other, with a summary of FAdV-8a challenged birds (strain 11-16629) in (A), and FAdV-8b challenged birds (strain 13-18153) in (B). The top panels show the neutralization against the virus type used for challenge, while the bottom panels show the neutralization against the respective heterologous virus type.

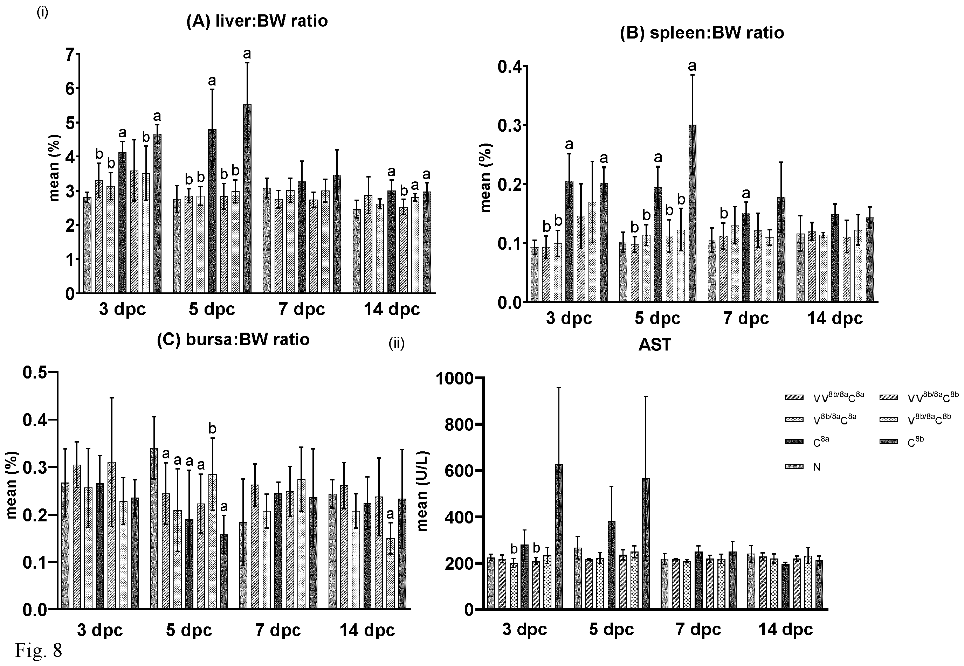

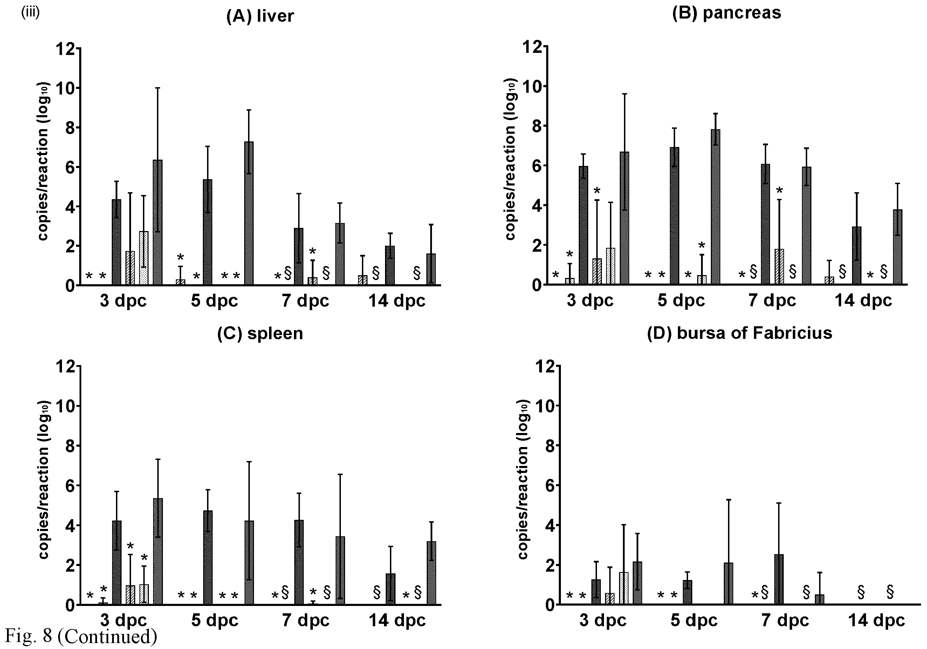

**Figure 8****.** Endpoints of protection in Protection study 2 (evaluated at 3, 5, 7 and 14 dpc). The groups are designated with V or VV, according to single or prime-boost regimen with crecFib-8b/8a, and C followed by the respective challenge virus (either FAdV-8a or FAdV-8b); N designates the negative control. (i) Organbody weight (BW) ratios for liver (A), spleen (B) and bursa of Fabricius (C). Lowercase letters above bars indicate significant differences (vs. negative control, "a"; vs. the corresponding challenge control, "b"). (ii) Plasma AST (significantly different vs. negative control, "a"; significantly different vs. challenge control, "b"). (iii) Viral load in liver (A), pancreas (B), spleen (C) and bursa of Fabricius (D). Significant differences vs. the corresponding challenge control are indicated with asterisks. Data exclude the single vaccination regimen at 7 and 14 dpc due to low sample size (marked with §).

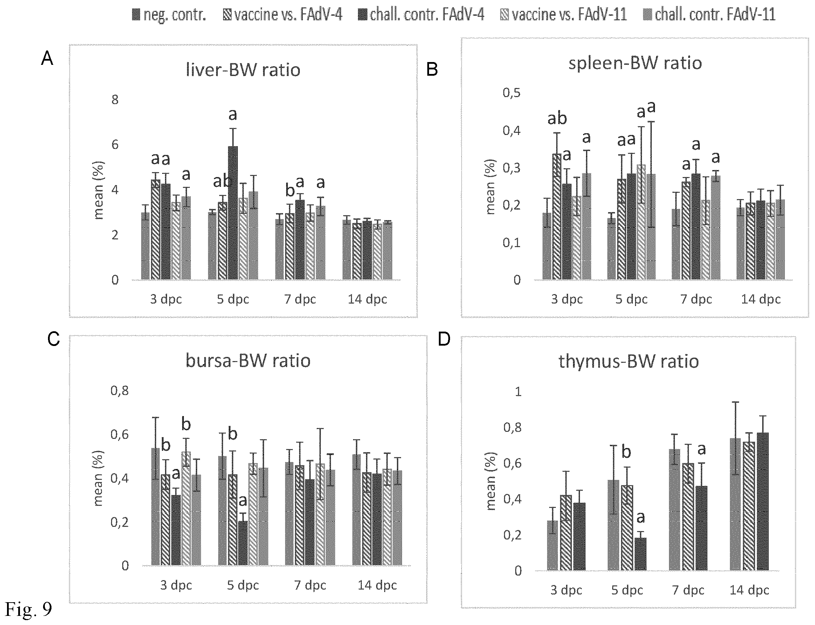

**Figure 9****.** Organ-BW mean values post-challenge. Mean and standard deviation of liver-BW ratio (A), spleen-BW ratio (B), bursa-BW ratio (C) and thymus-BW ratio (D) for each experimental group in the time period after challenge. Significant difference against negative control is indicated with letter "a", whereas significant difference against respective challenge control is indicated with letter "b". Significance was assessed at p ≤ 0.05. No significant difference was observed for thymus-BW in the FAdV-11 system at any measured time point.

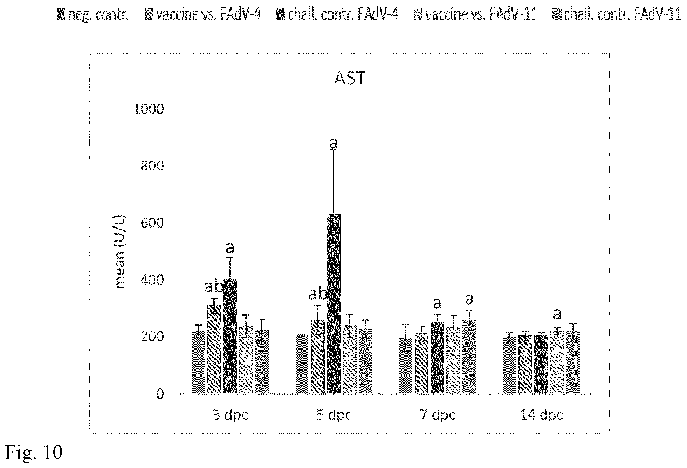

**Figure 10****.** AST mean values post-challenge. Mean and standard deviation of plasma AST for each experimental group in the time period after challenge. Significant difference against negative control is indicated with letter "a", whereas significant difference against respective challenge control is indicated with letter "b". Significance was assessed at p ≤ 0.05.

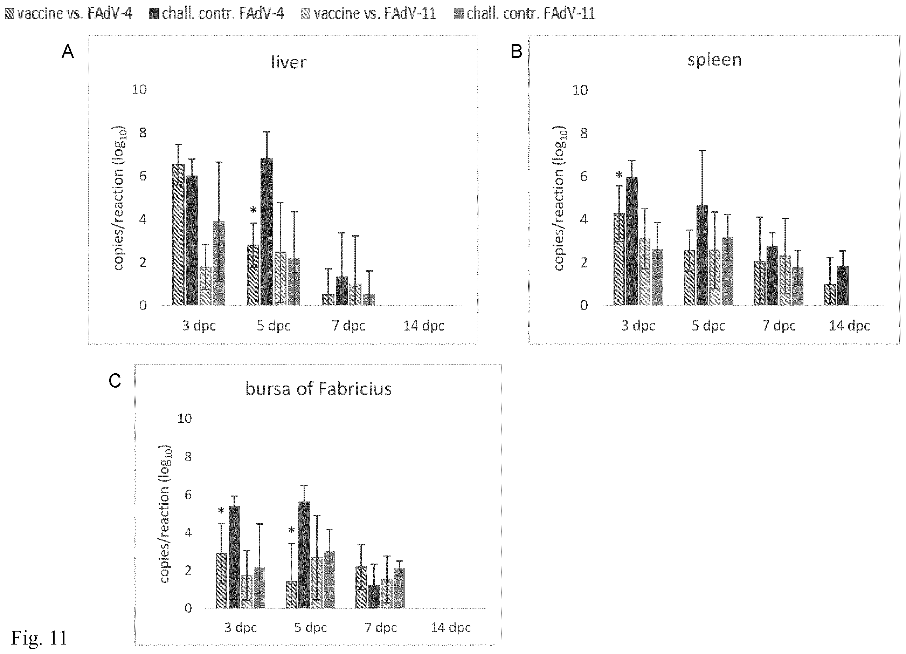

**Figure 11****.** Viral load in main target organs post-challenge. Mean and standard deviation of viral load (expressed in log10) for each experimental group in the time period after challenge in liver (A), spleen (B) and bursa of Fabricius (C). Asterisks indicate significant difference compared to the respective challenge control. Significance was assessed at p ≤ 0.05.

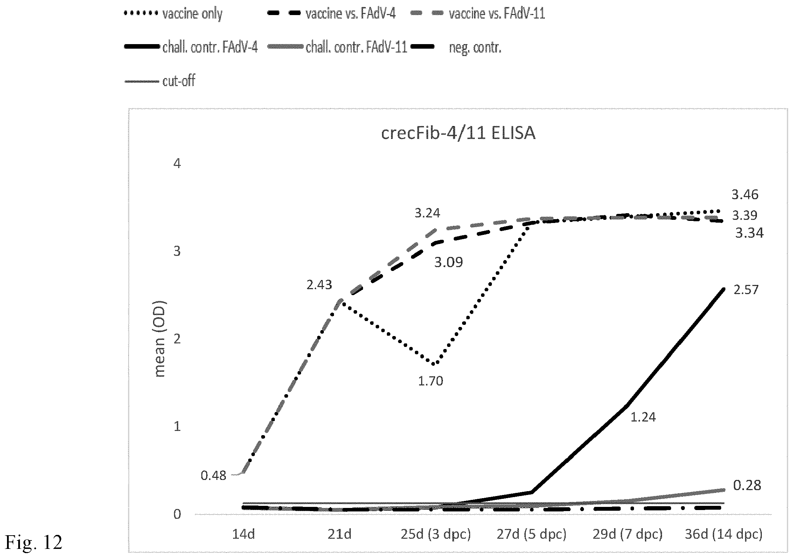

**Figure 12****.** Antibody development post vaccination. Mean titers (OD) for experimental groups tested on crecFib-4/11 ELISA throughout the protection study. Values given for the time point prior challenge (14 and 21d) are cumulative for the three vaccinated groups (I, II, III) and the two challenge control groups (IV, V). The cut-off value is marked in red.

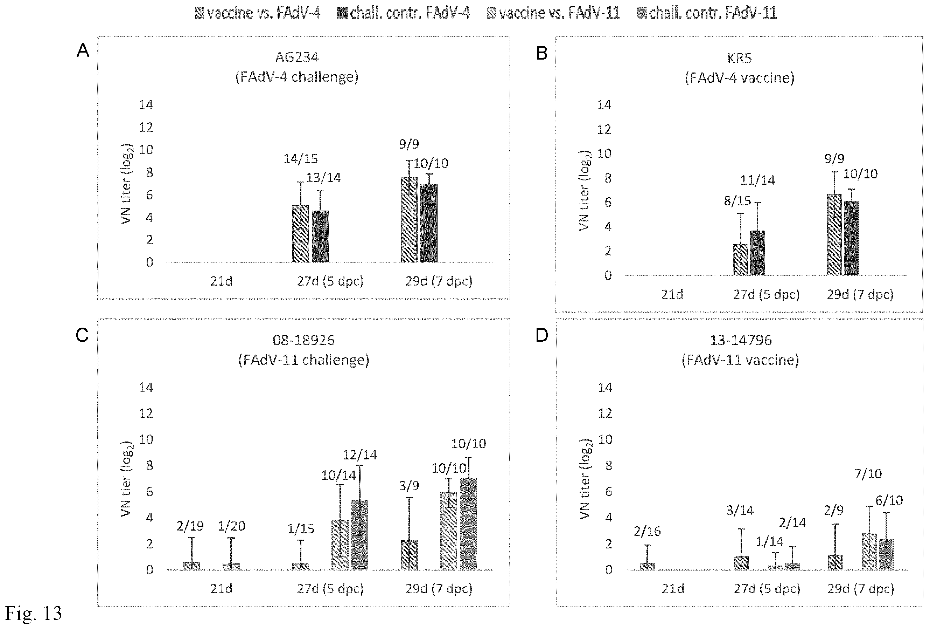

**Figure 13****.** Neutralizing antibodies development post vaccination. Mean and standard deviation of VNT titers (log2) before challenge (21d) and at 5-7 dpc: vaccinated groups and FAdV-4 challenge control against the FAdV-4 challenge (A) and vaccine (B) strain; vaccinated groups and FAdV-11 challenge control against the FAdV-11 challenge (C) and vaccine (D) strain. Headers show the number of birds exhibiting nAbs (some individuals could not be tested due to a lack of serum volume) . No nAbs were observed for the vaccine-only group at any measured time point.

### EXAMPLES

### Example 1: Cross-protective subunit vaccine with chimeric fiber based on two FAdV-E serotypes

The control of IBH, a disease of economic importance for chicken production worldwide, is complicated by an etiology involving multiple divergent FAdV types. The fiber protein is efficacious to induce protective antibodies in vaccinated chickens.

In this study, we designed chimeric proteins, swapping N- and C-distal fiber portions, each containing putative epitopes, between divergent serotypes FAdV-8a and -8b.

Remarkably, the chimeric fiber vaccine induced protective responses in chickens against both serotypes' infections.

### MATERIALS & METHODS

### In silico design for recombinantly expressed chimeric fibers ("crecFib")

The FAdV fiber open reading frame was divided into an amino (N)- and a carboxy (C)-distal segment, amplified separately from different template strains and fused seamlessly via Gibson assembly cloning to reconstitute a novel, full-length fiber, hereafter referred to as "crecFib" (Fig. 1a, 1b). As a result, crecFib constructs contain a crossover between heterologous sequences of serotypes FAdV-8a and -8b at the junction of N- and C-distal segments. The junction (termed "specificity switch") was engineered at consensus residue positions aa441-442 in the pairwise sequence alignment of the FAdV-8a and FAdV-8b fibers, mapping inside the proposed fiber head ("knob") domain. Accommodating *in silico* predicted epitopes to both sides, this strategy was anticipated to combine the antigenic specificity of both constitutive serotypes into a single antigen. By engineering the specificity switch inside the knob we also sought to maintain sequence integrity at the presumed shaft-knob boundary which represents an important element for trimerization of the fiber (Hong et al, 1996).

Candidate epitopes were (i) inferred from a previously reported epitope in the fiber-2 of FAdV-4 (FAdV-C) (Wang P et al, 2018), and (ii) *in silico* prediction with DiscoTope 2.0 software (Vindahl Kringelum et al, 2012), whereby the closest related fiber knob for which a molecular model is currently available, the fiber-2 of FAdV-1 (FAdV-A) reference strain CELO (El Bakkouri, 2008), served for homology modeling. Positional homologies between different type members were determined by multiple sequence alignments created with MegAlign software (DNASTAR, Madison, WI, USA). Homology modeling was also used for assigning structural domains (tail, shaft, knob) of the FAdV fiber based on existing information (Hess et al, 1995).

### Sequences of crecFib constructs

CrecFib-8a/8b:
   N-terminus:
      nt1-1323 from FAdV-8a reference strain ((TR59) KT862810) 
ATGGCGACCTCGACTCCTCACGCCTTCTCCTTTGGCCAAATCGGCTCCAGAAAACGCCCTGCGGGCGGCGATGGCGA
GCGAGACGCCTCCAAAGTGCCGAAAATGCAGACCCCCGCTCCGAGCGCGACCGCCAACGGAAATGACGAGCTGGACC
 
TGGTCTACCCCTTTTGGCTCCAAAACGGCTCCACCGGAGGAGGCGGCGGCGGTTCCGGTGGAAACCCGTCCCTC
AACCCGCCGTTTTTGGACCCCAACGGACCCCTGACCGTCCAAAACAACCTCCTGAAGGTCAATACCACGGCCCCCAT
CGCCGTCACCAATAAGGCCCTGACACTCTCCTTTGACGCGGAGAGTCTCGAGCTCACTAACCAACAGCAACTGGCGG
TCAAAATCGACCCCGAAGGACCTCTGAAAGCCACTACCGAGGGAATACAGCTGTCGGTCGACCCTACGACGTTGGAG
GTTGATGACGTCGACTGGGAGTTAACCGTGAACCTCGACCCCAACGGCCCCCTAGATTCCTCAGCCACAGGAATCAC
GGTCAGAGTCGATGACACCTTGCTCATCGATGATGACGGTTCCGGTCAAGGCAAAGAACTGGGCGTACATCTTAACC
CCGACGGACCCATTACGGCAGACCAAAACGGTCTCGACCTGGAAATCGACAACCAGACTCTTAAAATCACCCCCGGC
TCGGCGGGCGGTGTTCTTTCGGTACAGCTGAAACCGCAGGGAGGTCTGAATTCCCAATCCGATGGTATCCAGGTGGT
AACCCAGAACAGTATCGAAGTTGATAACGGCGCACTCGATGTTAAGGTAGTCGCTAACGGTCCACTGTCTACAACTC
CCAACGGTCTTACCCTAAACTACGATACCGGTGACTTCACCGTAAATGCCGGGACCTTAAGTATACTTCGGAACCCC
TCTCTCGTCGCCAATGCCTATCTTACGTCCGGGGCAAGAACACTGCAACAGTTCACCGCTAAAGGGGAAAACTCTAG
TCAGTTTTCCTTTCCATGTGCTTATTATCTTCAACAGTGGTTATCGGATGGACTCATTTTCAGTTCCCTCTACCTAA
AGTTAGATAGGACGCGCTTCACAGGGATGTCCAGCGATCCCTCCTATCAAAACGCCAGATATTTCACGTTCTGGGTA
GGGGGCGGCGCGGCTATGAATCTGTCCCAGTTAAGTACACCGACGATTACCCCCAGTACTACCGAGTGGACTGCATT
TGCTCCCGCCCTAAACTACTCCGGTGCGCCCGCCTTCGTTTATGATGCCAATCCAGTATTTACAATCTATTTCCAAC
CAAACACCGGGCGA
   C-terminus: nt1324-1569 from FAdV-8b reference strain ((764) KT862811) 
CTGGAAAGCTATCTCCCCGTCCTTACCGATAACTGGAGCCAAACCTACAACCCCGGCACCGTCACCCTGTGTGTAAA
AACGGTAAGGGTTCAATTGAGATCACAAGGAACCTTCAGCACTCTAGTCTGTTACAATTTCCGCTGTCAGAACACGG
GCATTTTTAACAGCAACGCTACAGCGGGAACCATGACACTTGGACCTATCTTCTTTAGTTGTCCTGCCTTAAGCACC
GCCAACGCTCCTTAA
   Complete sequence: 
ATGGCGACCTCGACTCCTCACGCCTTCTCCTTTGGCCAAATCGGCTCCAGAAAACGCCCTGCGGGCGGCGATGGCGA
GCGAGACGCCTCCAAAGTGCCGAAAATGCAGACCCCCGCTCCGAGCGCGACCGCCAACGGAAATGACGAGCTGGACC
TGGTCTACCCCTTTTGGCTCCAAAACGGCTCCACCGGAGGAGGCGGCGGCGGTTCCGGTGGAAACCCGTCCCTC
AACCCGCCGTTTTTGGACCCCAACGGACCCCTGACCGTCCAAAACAACCTCCTGAAGGTCAATACCACGGCCCCCAT
CGCCGTCACCAATAAGGCCCTGACACTCTCCTTTGACGCGGAGAGTCTCGAGCTCACTAACCAACAGCAACTGGCGG
TCAAAATCGACCCCGAAGGACCTCTGAAAGCCACTACCGAGGGAATACAGCTGTCGGTCGACCCTACGACGTTGGAG
GTTGATGACGTCGACTGGGAGTTAACCGTGAACCTCGACCCCAACGGCCCCCTAGATTCCTCAGCCACAGGAATCAC
GGTCAGAGTCGATGACACCTTGCTCATCGATGATGACGGTTCCGGTCAAGGCAAAGAACTGGGCGTACATCTTAACC
CCGACGGACCCATTACGGCAGACCAAAACGGTCTCGACCTGGAAATCGACAACCAGACTCTTAAAATCACCCCCGGC
TCGGCGGGCGGTGTTCTTTCGGTACAGCTGAAACCGCAGGGAGGTCTGAATTCCCAATCCGATGGTATCCAGGTGGT
AACCCAGAACAGTATCGAAGTTGATAACGGCGCACTCGATGTTAAGGTAGTCGCTAACGGTCCACTGTCTACAACTC
CCAACGGTCTTACCCTAAACTACGATACCGGTGACTTCACCGTAAATGCCGGGACCTTAAGTATACTTCGGAACCCC
TCTCTCGTCGCCAATGCCTATCTTACGTCCGGGGCAAGAACACTGCAACAGTTCACCGCTAAAGGGGAAAACTCTAG
TCAGTTTTCCTTTCCATGTGCTTATTATCTTCAACAGTGGTTATCGGATGGACTCATTTTCAGTTCCCTCTACCTAA
AGTTAGATAGGACGCGCTTCACAGGGATGTCCAGCGATCCCTCCTATCAAAACGCCAGATATTTCACGTTCTGGGTA
 
GGGGGCGGCGCGGCTATGAATCTGTCCCAGTTAAGTACACCGACGATTACCCCCAGTACTACCGAGTGGACTGCATT
TGCTCCCGCCCTAAACTACTCCGGTGCGCCCGCCTTCGTTTATGATGCCAATCCAGTATTTACAATCTATTTCCAAC
CAAACACCGGGCGACTGGAAAGCTATCTCCCCGTCCTTACCGATAACTGGAGCCAAACCTACAACCCCGGCACCGTC
ACCCTGTGTGTAAAAACGGTAAGGGTTCAATTGAGATCACAAGGAACCTTCAGCACTCTAGTCTGTTACAATTTCCG
CTGTCAGAACACGGGCATTTTTAACAGCAACGCTACAGCGGGAACCATGACACTTGGACCTATCTTCTTTAGTTGTC
CTGCCTTAAGCACCGCCAACGCTCCTTAA
CrecFib-8b/8a:
   N-terminus:
      nt1-1323 from FAdV-8b reference strain (764) KT862811) 
ATGGCGACCTCGACTCCTCACGCCTTCTCCTTTGGTCAAATCGGCTCCAGAAAACGCCCTGCGGGCGGCGATGGCGA
GCGAGACGCCTCCAAAGTGCCGAAAATGCAGACCCCCGCTCCGAGCGCGACCGCCAACGGAAATGACGAGCTGGACC
TGGTCTACCCCTTTTGGCTCCAAAACGGCTCTACCGGAGGAGGAGGCGGCGGTTCCGGTGGAAACCCGTCCCTC
AACCCGCCGTTTTTGGACCCCAACGGACCCCTGACCGTCCAAAACAACCTCCTGAAGGTCAATACCACGGCGCCCAT
CGCCGTCACCAATAAGGCCCTGACACTCGCCTATGAATCGGACAGTCTCGAGCTCACTAACCAGCAGCAACTGGCGG
TCAAAATCGACCCCGAAGGACCTCTGAAAGCCACTACCGAGGGAATACAGTTGTCGGTCGACCCTACGACGTTGGAG
GTTGATGACGTCGACTGGGAGTTGACGGTGAAACTCGACCCCGACGGCCCCCTGGATTCCTCAGCCACAGGAATCAC
GGTCAGAGTCGATGAGACCTTGCTCATCGAGGATGATGGGTCCGGTCAGGGCAAAGAACTCGGAGTCAATCTCAACC
CCACCGGACCGATTACGGCCGATGACCAGGGTCTGGACTTAGAAATAGACAACCAGACGCTCAAGGTCAACAGTGTT
ACCGGCGGGGGCGTCCTAGCTGTACAACTCAAATCCCAAGGTGGTCTTACCGCACAGACTGACGGTATCCAAGTGAA
CACTCAGAACAGCATCACCGTTACAAACGGAGCTCTGGACGTGAAAGTAGCCGCCAACGGACCTTTGGAGTCAACCG
ACACCGGGCTCACACTCAACTATGACCCCGGAGACTTCACAGTTAATGCGGGCACGTTGAGCATTATCAGGGATCCG
GCTCTCGTGGCCAATGCGTACCTCACATCCGGCGCCTCCACCCTTCAGCAATTTACAGCTAAGAGTGAAAATTCCAG
TCAATTTTCTTTCCCATGCGCATACTATCTGCAACAGTGGCTTTCCGATGGGTTGATTTTTAGCTCCCTCTATCTGA
AGCTCGACAGAGCACAGTTCACGAACATGCCAACGGGTGCAAATTATCAGAACGCCAGGTACTTTACCTTCTGGGTT
GGAGCGGGCACTTCATTTAATCTTTCTACCCTTACCGAACCCACTATTACACCCAACACCACACAATGGAATGCATT
CGCCCCTGCCCTTGATTACTCAGGTGCTCCTCCCTTCATCTACGACGCGTCTTCCGTAGTTACGATTTATTTTGAAC
CCACCAGTGGTCGA
   C-terminus:
      nt1324-1575 from FAdV-8a reference strain ((TR59) KT862810) 
GTGGACACATACCTGCCGGTTCTCACCGGCGACTGGAAAACTTCTTCCACTTACAATCCAGGAACAATAACACTCGT
CGTAAGGAACGCTACGATCCAGCTGCAATCACAGAGCACGTTCACAACCAGTGTGTGTTACAACTTCCGATGTCAAA
ACTCTGGGATTTTCAACAACAACGCCACCTCGGGAACGCTCACCCTAGGTCCGATCTTTTACAGTTGTCCGGCCCTA
AGTACTGCGGACGTGTCATAA
   Complete sequence:

ATGGCGACCTCGACTCCTCACGCCTTCTCCTTTGGTCAAATCGGCTCCAGAAAACGCCCTGCGGGCGGCGATGGCGA
GCGAGACGCCTCCAAAGTGCCGAAAATGCAGACCCCCGCTCCGAGCGCGACCGCCAACGGAAATGACGAGCTGGACC
TGGTCTACCCCTTTTGGCTCCAAAACGGCTCTACCGGAGGAGGAGGCGGCGGCGGTTCCGGTGGAAACCCGTCCCTC

AACCCGCCGTTTTTGGACCCCAACGGACCCCTGACCGTCCAAAACAACCTCCTGAAGGTCAATACCACGGCGCCCAT
CGCCGTCACCAATAAGGCCCTGACACTCGCCTATGAATCGGACAGTCTCGAGCTCACTAACCAGCAGCAACTGGCGG
TCAAAATCGACCCCGAAGGACCTCTGAAAGCCACTACCGAGGGAATACAGTTGTCGGTCGACCCTACGACGTTGGAG
GTTGATGACGTCGACTGGGAGTTGACGGTGAAACTCGACCCCGACGGCCCCCTGGATTCCTCAGCCACAGGAATCAC
GGTCAGAGTCGATGAGACCTTGCTCATCGAGGATGATGGGTCCGGTCAGGGCAAAGAACTCGGAGTCAATCTCAACC
CCACCGGACCGATTACGGCCGATGACCAGGGTCTGGACTTAGAAATAGACAACCAGACGCTCAAGGTCAACAGTGTT
ACCGGCGGGGGCGTCCTAGCTGTACAACTCAAATCCCAAGGTGGTCTTACCGCACAGACTGACGGTATCCAAGTGAA
CACTCAGAACAGCATCACCGTTACAAACGGAGCTCTGGACGTGAAAGTAGCCGCCAACGGACCTTTGGAGTCAACCG
ACACCGGGCTCACACTCAACTATGACCCCGGAGACTTCACAGTTAATGCGGGCACGTTGAGCATTATCAGGGATCCG
GCTCTCGTGGCCAATGCGTACCTCACATCCGGCGCCTCCACCCTTCAGCAATTTACAGCTAAGAGTGAAAATTCCAG
TCAATTTTCTTTCCCATGCGCATACTATCTGCAACAGTGGCTTTCCGATGGGTTGATTTTTAGCTCCCTCTATCTGA
AGCTCGACAGAGCACAGTTCACGAACATGCCAACGGGTGCAAATTATCAGAACGCCAGGTACTTTACCTTCTGGGTT
GGAGCGGGCACTTCATTTAATCTTTCTACCCTTACCGAACCCACTATTACACCCAACACCACACAATGGAATGCATT
CGCCCCTGCCCTTGATTACTCAGGTGCTCCTCCCTTCATCTACGACGCGTCTTCCGTAGTTACGATTTATTTTGAAC
CCACCAGTGGTCGAGTGGACACATACCTGCCGGTTCTCACCGGCGACTGGAAAACTTCTTCCACTTACAATCCAGGA
ACAATAACACTCGTCGTAAGGAACGCTACGATCCAGCTGCAATCACAGAGCACGTTCACAACCAGTGTGTGTTACAA
CTTCCGATGTCAAAACTCTGGGATTTTCAACAACAACGCCACCTCGGGAACGCTCACCCTAGGTCCGATCTTTTACA
GTTGTCCGGCCCTAAGTACTGCGGACGTGTCATAA

### Cloning and expression of crecFib constructs

Based on FAdV reference strains, selected as expression templates, the above defined N- and C-distal fiber segments were amplified using primer pairs featuring overhangs with the flanking sequence of the EheI/StuI-digested pFAST BAC expression vector (Invitrogen, Vienna, Austria) and between the two segments themselves. Ligation into the linearized pFAST BAC vector was performed with the Gibson Assembly Master Mix (NEB, Ipswich, MA) according to the manufacturer's instructions. Two constructs were generated for each chimeric combination, with reciprocal specificity order, designated accordingly as crecFib-8a/8b and crecFib-8b/8a.

Correct insertion of the segments into the vector was confirmed by Sanger sequencing across the multiple cloning site (LGC Genomics, Berlin, Germany).

Recombinant proteins were expressed in *Spodoptera frugiperda* Sf9 cells and purified via the polyhistidine tag on affinity chromatography columns as described previously (Schachner et al, 2014), and their concentration determined by Bradford assay (Thermo Fisher Scientific, Vienna, Austria).

### Assessment of the immunogenicity and in vitro reactivity spectrum of crecFib

Specific pathogen-free (SPF) chickens (Valo BioMedia GmbH, Osterholz-Scharmbeck, Germany), hatched and housed at our facilities, were immunized intramuscularly (i.m.) with crecFib-8a/8b (n=5) or crecFib-8b/8a (n=5). Three birds of each group received 50 µg, two birds 100 µg recombinant protein mixed 1:1 with GERBU adjuvant P (GERBU Biotechnik GmbH, Heidelberg, Germany). Post-immunization sera were collected in weekly intervals for parallel monitoring by ELISA and virus neutralization test.

Additional immune sera for comparative purposes were provided. Briefly, these sera were derived from SPF chickens injected with whole virus or immunized i.m. with monospecific FAdV fibers (i.e., recombinantly expressed fibers based on the sequence of a singular serotype). Whole virus for antiserum production was 3-fold plaque-purified and characterized at least by partial analysis of the hexon and fiber genes, but in most cases by fullgenome sequencing, and cross neutralization test, as described earlier (Schachner et al, 2016). Whenever available, our test setting included sera against inactivated, adjuvanted FAdV (prepared with 1% formaldehyde, administered in a 1:1 mixture with GERBU adjuvant) and live virus, in order to assess a possibly differential recognition of denatured antigen in the immunoblot. crecFib-based enzyme-linked immunosorbent assay (ELISA)

Sera from birds immunized with each type of crecFib were tested on ELISA plates coated with the corresponding crecFib, following the protocol described by Feichtner et al., 2018. Virus (cross) neutralization (VN) test

Neutralizing antibodies (nAbs) in sera were determined in a microtiter assay on primary chicken-embryo liver (CEL) cells. Serial 2-fold serum dilutions (1:8 - 1:16,384) were incubated with 100 TCID50 of virus. The crecFib antisera were tested against the FAdV-8a and -8b reference (expression template) strains and two field isolates from each serotype which served as challenge strains in the protection study. For comparative purposes, antifiber sera with monotype specificity (Fib-8a and Fib-8b serum) were included in certain settings. Additionally, each microtiter plate included positive (cells + virus) and negative (only cells) control wells. After five days at 37 °C in 5% CO2, the wells were investigated for cytopathic effect (CPE), with the titer defined by the highest serum dilution inhibiting CPE.

### Immunofluorescence (IF) staining

Following a virus cross-neutralization test against FAdV-8a and -8b reference strains, as described above, the cells were fixed by adding ice-cold methanol for 5 min. Following serial washing steps and blocking with 3% BSA for 1 h, in-house generated polyclonal rabbit α-FAdV (diluted 1:500 in PBS) was added to each well overnight. After removal of the rabbit antiserum and washing, the plates were incubated with 1:200 diluted Alexa Fluor 488-conjugated Donkey anti-Rabbit IgG (Invitrogen, Life Technologies, Carlsbad, CA, USA) in the dark for 1 h. After another wash, cells were stained with 1:1000 4'6-diamidino-2-phenylindole solution (DAPI; Roche Diagnostics GmbH, Vienna, Austria) for 5 min, subjected to a final wash and covered with PBS.

Internalization of viral particles was examined and documented with a Zeiss Axiovert 200 M fluorescence microscope (Zeiss, Jena, Germany), coupled to a FLEXACAM C1 camera and the Leica Application Suite X (LAS X) (Leica Microsystems GmbH, Wetzlar, Germany).

### Western blot

The crecFib constructs were screened side-by-side with the closest related, in-house expressed monospecific fibers (Fib-8a/strain TR59, Fib-8b/strain 764 and Fib-7/strain YR36, the reference type representatives of FAdV-E) using polyclonal immune sera. In order to minimize variations in the ratio of reactants, the concentration of recombinant protein loaded per lane was adjusted to 7.5 µg, and detection sera with similar ELISA titers (2.5 ≤ OD ≤ 3.0) against recombinant fiber of the homologous type and, if applicable, neutralization titers in the range of log211-12 were used. Briefly, recombinant purified proteins were separated by 12% SDS-PAGE and transferred onto BioTrace PVDF Transfer Membrane (Pall, Vienna, Austria) with the Trans-Blot Turbo Transfer System (Bio-Rad, Vienna, Austria). After blocking with 3% (w/v) skim milk, membranes were incubated separately with polyclonal sera, preabsorbed with 1% Sf9 cell powder and diluted 1:2000. As a control for presence and size of monomeric fibers, one membrane was incubated with anti-polyhistidine antibody (Sigma-Aldrich, Vienna, Austria). Following incubation with secondary Rabbit-Anti-Chicken-IgG-HRP (Sigma-Aldrich, Vienna, Austria) or Goat-Anti-Mouse-IgG(H + L)-HRP (Bio-Rad, Vienna, Austria) for controls, and intermediate washes, blots were developed with Clarity Western ECL substrate (Bio-Rad, Vienna, Austria). Visualization was performed with the ChemiDoc Imager (Bio-Rad, Vienna, Austria).

### Protection studies with crecFib constructs

Two vaccination-challenge trials were performed to sequentially address whether (i) crecFib constructs confer *in vivo* protection, and (ii) crecFib-induced protection is amenable for broad coverage of the IBH complex. An overview of both experimental designs is summarized in Fig. 4.

### Clinical trial 1: Protective efficacy of crecFib-8a/8b and crecFib-8b/8a

In the first study, two groups of SPF broiler chickens (n=12) were prime-boost vaccinated with either crecFib-8a/8b or the reverse-order crecFib-8b/8a, followed by challenge with FAdV-8b in each case. SPF broilers were obtained from Animal Health Service (Deventer, The Netherlands) and housed in separate isolator units (HM2500, Montair, The Netherlands). Vaccination consisted of 50 µg of the respective crecFib formulated in a 40% (w/v) antigen-oil-based adjuvant phase, administered i.m., while challenge was carried out i.m. with 106.2 TCID50 FAdV-8b (strain 13-18153). Further groups served as challenge control, injected with a PBS/adjuvant mixture instead of vaccination, and negative control, administered only sterile PBS according to the same scheme. Blood was collected weekly from booster until challenge, and at 3, 5, and 7 days post challenge (dpc). Four birds per group were killed and submitted to necropsy at 3 and 5 dpc, analogous to the remaining birds at 7 dpc. Endpoints of protection included clinical signs, assessed daily in the time period post challenge, organbody weight (BW) ratios for liver and spleen, the aspartate transaminase (AST) content in plasma as previously described (Matos et al, 2016), as well as viral load quantification in liver and pancreas by a qPCR protocol adapted from Gunes et al., 2012.

### Clinical trial 2: Broad-protective efficacy of crecFib-8b/8a applying different vaccination regimens

In this setting, we proceeded with only one of the chimeras, crecFib-8b/8a, this time testing its protective efficacy against challenge with both viral types of interest (FAdV-8a or FAdV-8b). Additionally, a prime-boost vaccination regimen was compared to a single-shot regimen, using groups of 20 SPF broilers.Each vaccination contained 50 µg crecFib-8b/8a formulated in a 40% (w/v) antigen-oil-based adjuvant phase, administered i.m. Challenge was carried out i.m. with 106.2 TCID50 of FAdV-8a (strain 11-16629) or FAdV-8b (strain 13-18153), while negative control birds again received PBS instead.

Blood was collected weekly from the second week of life until challenge, then at each of the following sampling time points: 3, 5, 7 and 14 dpc. Up to five birds/group were killed and necropsied at 3, 5, 7 and 14 dpc alongside individuals that died due to the infection.

Endpoints of protection included organ-BW ratios for liver, spleen and bursa of Fabricius, the AST content in plasma, and viral load in liver, pancreas, spleen, and bursa of Fabricius.

Statistical analysis of the datasets was carried out using Shapiro-Wilk test together with a visual inspection of histograms and normal Q-Q plots in order to verify the normal distribution assumption. The mean values for organ-BW ratios, plasma AST and viral load in target organs of vaccinated groups were compared with the negative control and their corresponding challenge control groups via unpaired Student's t-test. Datasets which did not meet the normality assumptions were analyzed through pairwise comparisons with Mann-Whitney U test. In each case, p values ≤0.05 were considered statistically significant. Statistical analyses were performed with SPSS Version 26 (IBM SPSS Statistics; IBM Corp., Armonk, NY, USA).

### RESULTS

### In silico design and recombinant expression of crecFib constructs

*In silico* epitope analysis of the TR59 and 746 fiber knobs suggested sites that - based on homology modeling - were assigned to the CD loop (G.SSD, N.PTG), the β-strand F/FG loop (V.DANP, I.DASS), and the HI loop of the knob (QSQ, RSQ). According to three-dimensional models created on basis of the chimeric knobs' sequences all predicted epitopes were localized externally as well as apically on the molecule (Fig. 1c). Furthermore, the two sites in β-strand F/FG loop and HI loop, though non-contiguous in the primary sequence, were conformationally in contact, revealing a coherent surface patch formed by residues encoded N- and C-distally of the chimeric junction.

Both chimeric proteins were successfully recovered from the soluble fraction of infected Sf9 cells, as confirmed by bands with the appropriate monomer size in western blot. The yields of purified chimeric fibers were approximately 13.4 (crecFib-8a/8b) and 14.8 mg per liter Sf9 cell culture (crecFib-8b/8a).

### Immunogenicity and in vitro reactivity spectrum of crecFib crecFib antibody induction detected by ELISA

Based on the homologous antigen-ELISA, birds immunized with crecFib-8a/8b showed a flat increase in OD magnitude, with only sporadic occurrence of ODs>1 in a single individual at three successive timepoints from 5-7 weeks post vaccination (wpv). All other birds did not exceed OD 0.5 at any timepoint during an 8-week-monitoring period (Fig. 2). In contrast, birds immunized with the reverse-order crecFib-8b/8a developed a sharp rise in OD on the homologous ELISA antigen as early as 2 wpv, at which 5/5 birds already presented with ODs close to 3 (mean OD 3.00±0.18), independent of the antigen dose (50 vs. 100 µg). The crecFib-8b/8a ODs remained at a constant level in all five individuals until the end of the monitoring period at 8 wpv, with mean weekly ODs in the range of 2.76±0.29 to 3.39±0.12.

### Neutralizing activity of crecFib antisera

In crecFib-8b/8a antisera, neutralization was first present at 2 wpv, with all birds showing low to moderate titers (log₂4-7) against at least one of the constitutive types (Fig. 2). In fact, already 4/5 individuals had developed bilateral nAbs against both types at this timepoint. Neutralizing titers of the crecFib-8b/8a antisera continuously increased during the monitoring period; from 5 wpv onwards all birds had titers ≥log₂6 against the complete set of tested strains with one bird reaching the maximum of the measured range (log₂14) against FAdV-8b. On an individual bird level, the neutralizing responses showed an overall balanced distribution, with similar titers against both types, regardless whether the corresponding virus was a template (reference) or field strain.

Immunofluorescent staining of a virus neutralization setting, which directly compared crecFib-8b/8a antiserum side-by-side with antifiber sera against Fib-8a or Fib-8b on the same microtiter plate, demonstrated that only the chimeric serum could efficiently inhibit the infection of both FAdV-8a (strain TR59) and -8b (strain 764) *in vitro.* While the monospecific Fib-8a and Fib-8b antisera exerted neutralizing activity against their cognate serotypes at similar titers compared to the crecFib antiserum, neither one of them could inhibit the opposite serotype.

### Recognition of fibers in western blot

All investigated recombinant fibers, independent of their genetic background and their monospecific or chimeric composition, were detected by immune sera representing the complete spectrum of FAdV types, defined by fiber specificity (Fig. 3).

Mutual recognition was even possible between reaction partners with differential fiber expression (fiber-1, fiber-2 of FAdV-A and FAdV-C vs. singular fiber of the remaining FAdV species), and between chimeric counterparts with reverse template order (e.g. crecFib-8b/8a antiserum was able to recognize the crecFib-8a/8b) .

Of note, no differences were noted between antisera raised against live (native) FAdV and inactivated, adjuvant-formulated virus preparations, as well as subunit-directed sera, in their ability to recognize the fiber monomer in immunoblots.

### Clinical trials

### Clinical trial 1: Protective efficacy of crecFib-8a/8b and crecFib-8b/8a

An overview of the *in vivo* experimental designs is provided in Fig. 4. A group designated VV^{8a/8b}C^{8b}, according to the treatments carried out, was vaccinated twice with crecFib-8a/8b at 3 and 21 days of life, followed by FAdV-8b challenge at 36 days of life (=15 days post booster, dpb). A second group designated VV^{8b/8a}C^{8b} was vaccinated at the same timepoints with the reverse-order crecFib-8b/8a, and then also challenged with FAdV-8b. The challenge control, mock-vaccinated with a PBS/adjuvant mixture prior to FAdV-8b challenge, is designated as C^{8b}, and the negative control, administered only sterile PBS at each occasion, as N.

Onset of vaccine-induced antibody development was not detected by ELISA until after booster (7 dpb=28 days of life), although only at a low level indicating a beginning rise in birds of VV^{8a/8b} (mean OD 0.51±0.65), while peak detectable levels were already reached in VV^{8b/8a} (3.14±0.73) (Fig. 5). In comparison, birds of N and C^{8b} (prior to challenge) never exceeded OD 0.07±0.01. Immediately before challenge (14 dpb) VV^{8a/8b} reached moderate ODs (1.45±0.95), but none of the sera exhibited neutralizing activity (SN titers ≤log₂8). In VV^{8b/8a} OD levels remained rather constant (2.60±0.85) and, with exception of a single bird, all sera exhibited neutralizing activity, ranging from low titers against at least one of the tested serotypes (in 3 birds) to titers at the highest end of the measured range (log₂14) against both serotypes (FAdV-8a and -8b reference and challenge viruses) in the remaining birds. As a general trend, higher titers (and earlier onset, based on those sera with only unilateral neutralization) were noted against FAdV-8a; in addition, nAbs against FAdV-8b never diverged by more than 3 titer levels between reference and challenge strain, while nAbs against FAdV-8a diverged up to 5 titer levels with more pronounced reactivity against the challenge, compared to the reference strain.

Following challenge, mild depression (inappetence, huddling, ruffled plumage) was recorded in two birds of C^{8b} at 4-5 dpc. During necropsy at this timepoint, the same individuals had significantly increased liver:BW ratios. An increase of liver:BW ratios was also recorded in VV^{8a/8b}C^{8b} and VV^{8b/8a}C^{8b}, but occurred delayed at 7 dpc (Fig. 6). The C^{8b} group additionally experienced a significant increase of the spleen:BW (7 dpc), which was absent in VV^{8b/8a}C^{8b}, whereas moderate, yet not significant, in VV^{8a/8b}C^{8b}.

Highest plasma AST was recorded in C^{8b} at 3 and 5 dpc, being significant at 5 dpc, whereas VV^{8a/8b}C^{8b} and VV^{8b/8a}C^{8b} remained comparable to N (Fig. 6) . At 7 dpc plasma AST values were not distinguishable between the groups anymore.

A reduction of hepatic viral load vs. C^{8b} was noted in both VV^{8a/8b}C^{8b} and VV^{8b/8a}C^{8b} at all investigated timepoints, with consistently lowest values and significance at 5 dpc in VV^{8b/8a}C^{8b} (Fig. 6). In the pancreas, viral DNA was completely absent in VV^{8b/8a}C^{8b} at 3 dpc and significantly reduced at 5 and 7 dpc. In VV^{8a/8b}C^{8b} pancreatic viral load was significantly reduced at 5 dpc.

### Clinical trial 2: Broad-protective efficacy of crecFib-8b/8a applying different vaccination regimens

A prime-boost vaccination regimen at day-old and 7 dpv was administered to two groups, one of which was challenged at 22 days of age with FAdV-8a (designated VV^{8b/8a}C^{8a}), the other with FAdV-8b (VV^{8b/8a}C^{8b}). Additionally, this was compared to a single-vaccination regimen at day-old (groups V^{8b/8a}C^{8a}, V^{8b/8a}C^{8b}). Mock-vaccinated challenge control groups were included for each challenge type (C^{8a}, C^{8b}), and a negative control group (N) receiving PBS analogous to the prime-boost regimen.

At 21 days of life, immediately prior challenge, birds of each vaccination regimen had developed antibody levels detectable by ELISA. However, the prime-boost group (VV^{8b/8a}) had higher ODs with only 1/40 individuals having OD <3 (mean OD 3.3±0.1), while the single-vaccine regimen (V^{8b/8a}) had overall lower, more unevenly distributed, titers (2.65±0.79) (Fig. 7). Post-challenge and irrespective of the challenge virus type, ODs between V^{8b/8a} and VV^{8b/8a} were not distinguishable anymore, as they reached (and then remained at) the highest measurable endpoint. In contrast, nonvaccinated challenge control birds showed a steady incline of titers to 2.15±1.02 until 14 dpc (C^{8a}), or an abrupt rise to 2.02±0.95 at 7 dpc remaining stable thereupon (C^{8b}).

In 36/40 VV^{8b/8a} birds pre-challenge Abs had neutralizing activity against both FAdV-8a and -8b, while the remaining four birds showed only unilateral neutralizing activity against one of the serotypes. In comparison, single-vaccinated V^{8b/8a} birds showed a more infrequent presence of pre-challenge nAbs, with 11/40 birds with unilateral nAbs, and seven birds with complete absence of nAbs. Furthermore, titer levels were lower in V^{8b/8a} compared to VV^{8b/8a}, although the regimens were similar in eliciting stronger neutralization against FAdV-8a (log₂3.28±3.22 in V^{8b/8a} vs. log₂5.58±2.00 in VV^{8b/8a} against FAdV-8a, log₂2.88±2.44 in V^{8b/8a} vs. log₂4.30±3.13 in VV^{8b/8a} against FAdV-8b).

The FAdV-8a challenge resulted in a relatively abrupt increase of nAbs in naive birds (C^{8a}), reaching higher levels compared to any of the vaccinated groups at 7 dpi. In VV^{8b/8a}C^{8a} overall higher titers against FAdV-8b than FAdV-8a were found, while the reverse trend was seen in v^{8b/8a}C^{8a}; by 14 dpc birds of all three groups reached comparable mean titers against FAdV-8a. FAdV-8b challenge induced similar mean titers in naïve birds (C^{8b}) than FAdV-8a challenge at 7 dpc (log₂10.70±1.34), but did not significantly exceed the mean titers of vaccinated birds (V^{8b/8a}C^{8b}, VV^{8b/8a}C^{8b}). Although V^{8b/8a}C^{8b} and VV^{8b/8a}C^{8b} continued to develop nAbs against FAdV-8a, mean titers against FAdV-8b were still always higher.

As benchmark for clinical affection following challenge, mild depression was recorded in one (C^{8a}) and four birds (C^{8b}) between 2-5 dpc, and an additional dead bird at 4 dpc in C^{8b}. Among all birds of the vaccinated groups, only one case of transient depression occurred at 2-3 dpc, less surprisingly in an individual of V^{8b/8a}C^{8b} lacking pre-challenge nAbs.

Mean liver- and spleen:BW ratios were most affected at 3 and 5 dpc with significant increase in both challenge controls (Fig. 8). Despite levelling off, this effect still persisted for the spleen at 7 dpc in C^{8a}, and for the liver at 14 dpc in both C^{8a} and C^{8b}. In contrast, liver- and spleen:BW ratios of vaccinated/challenged groups remained comparable to the negative control (N) throughout the whole experiment, except for the liver of V^{8b/8a}C^{8b} at one timepoint (14 dpc). Bursa:BW ratios were most affected at 5 dpc, with a significant reduction vs. N in all groups, except for V^{8b/8a}C^{8b} in which the same trend was shifted to 14 dpc.

In addition, C^{8a} and C^{8b} had the highest plasma AST levels of all groups from 3-7 dpc; significant differences were found with two of the vaccinated groups (VV^{8b/8a}C^{8b} and V^{8b/8a}C^{8a}). Furthermore, vaccinated groups exhibited consistently lower viral loads in target organs compared to their challenge controls, while vaccination even prevented detectable infection at several timepoints. With few exceptions, liver, pancreas and spleen samples of C^{8a} and C^{8b} were positive at all timepoints (only one liver at 7 dpc, and pancreas and spleen from another bird at 14 dpc in C^{8a} were negative; as well as two livers at 14 dpc in C^{8b}); in contrast, viral DNA was detected only in one bird's liver at 5 dpc, and another bird's liver and pancreas at 14 dpc in VV^{8b/8a}C^{8a}, with viral loads significantly reduced from 3-7 dpc. Similar results were achieved in V^{8b/8a}C^{8a} between 3-5 dpc, with only one bird's pancreas and spleen tested positive at the earliest timepoint (3 dpc). In VV^{8b/8a}C^{8b} mean viral loads were significantly reduced at 5-7 dpc in liver, and at all timepoints in pancreas and spleen; in fact, all of the target organs remained even completely negative at 5 and 14 dpc. In V^{8b/8a}C^{8b}, a significant reduction of viral load occurred in the spleen at 3-5 dpc, and in liver and pancreas at 5 dpc. The bursa of Fabricius was the organ with the lowest mean viral load, although positives were still found in all but two samples in C^{8a}, and in 4, 3 and 1 sample (s) in C^{8b} from 3-7 dpc. Of all vaccinees, only three individuals, having no pre-challenge nAbs and challenged with FAdV-8b, were positive at 3 dpc. At 14 dpc viral DNA was generally not detectable in the bursa anymore.

### Example 2: Vaccination with an FAdV chimeric fiber protein simultaneously protects chickens against HHS and IBH

In the past decades, FAdV-related diseases have become an increasing concern for poultry industry worldwide. Various immunization strategies against FAdVs have been experimentally investigated, with a particular focus on subunit vaccines against HHS, caused by FAdV serotype 4, and IBH, caused by serotypes 2, 8a, 8b and 11. In this study, we extended the innovative concept of recombinant chimeric fiber proteins to design a novel chimera retaining epitopes from serotypes belonging to both FAdV-4 and - 11, and we investigated its efficiency to simultaneously protect chickens against HHS and IBH. Specific pathogen-free chickens were vaccinated with the novel chimeric fiber and subsequently challenged with either an HHS- or IBH-causing strain. The development of neutralizing antibodies was limited against FAdV-11 and absent against FAdV-4. Surprisingly, vaccinated birds nevertheless exhibited a reduction of clinical signs, limited hepatomegaly and lower levels of AST compared to the respective challenge controls upon viral challenge. Furthermore, the vaccine prevented atrophy of HHS-affected lymphoid organs, such as thymus and bursa of Fabricius, and viral load in the target organs was significantly reduced. Clinical protection was associated with high level of pre-challenge antibodies measured on ELISA plates coated with the vaccination antigen. In conclusion, we proved that the concept of chimeric fiber vaccines can be extended across viral species boundaries. Our vaccine represents the first FAdV subunit vaccine able to achieve comprehensive protection against different FAdV-associated diseases.

### MATERIALS AND METHODS

### Design and expression of chimeric fiber

A chimeric fiber protein retaining epitopes from FAdV-4 (fib-2) and FAdV-11 was designed in this study. Briefly, the fiber open reading frame (ORF) was divided into an amino (N)- and a carboxy (C)-distal segment, amplified separately from different template strains and fused seamlessly via Gibson assembly cloning to reconstitute a novel, full-length fiber. Representing a crossover between heterologous sequences of discrete serotypes, the junction of N- and C-distal segments is hereafter referred to as specificity switch. The switch was engineered at an intertype consensus motif corresponding to positions aa441-442 or aa491-492 in the pairwise sequence alignments of FAdV-4 fiber-2 and FAdV-11 fiber, which map to the proposed G-strand inside the fiber head ("knob") domain. Accommodating putative B cell epitopes to both sides, this strategy was allowed incorporation of epitopic regions from both constitutive serotypes into the final chimeric product. Additionally, the knob-internal modification could also be reconciled with maintenance of sequence integrity at the presumed shaft-knob boundary which has a reported function in trimerization of the fiber (Hong & Engler, 1996). Candidate epitopes were inferred from a previously reported epitope in the fiber-2 of FAdV-4 (Wang et *al.,* 2018) and *in silico* prediction with DiscoTope 2.0 software (Vindahl Kringelum et *al.,* 2012) utilizing the closest related fiber for which a molecular model is currently available, the fiber-2 of FAdV-1 reference strain CELO (El Bakkouri et *al.,* 2008). Subsequently, positional homologies between different type members were determined on basis of multiple sequence alignments created with MegAlign software (DNASTAR, Madison, WI, USA). Homology mapping was also used for assigning structural domains (tail, shaft, knob) of the FAdV fiber based on existing information (Hess et *al.,* 1995). Based on FAdV reference strains selected as expression templates the N- and C-distal fiber segments, as defined above, were amplified using primer pairs featuring overhangs with the flanking sequence of the *EheI*/*StuI*-digested pFAST BAC expression vector (Invitrogen, Vienna, Austria) and between the two segments themselves. Detailed information on the cloning strategy is provided in Table 1. Ligation into the linearized pFAST BAC vector was performed with the Gibson Assembly Master Mix (NEB, Ipswich, MA) according to the manufacturer's instructions and the resulting construct was named crecFib-4/11.

**Table 1. Chimeric FAdV fiber protein used in this study. Overhangs at the 5'-termini of primer sequences with the flanking vector sequence (FP1 and RP2 primers) or with the counterpart template sequence (RP1 and FP2 primers) are represented by underlined nucleotides.**

| **Designation of chimeric construct** | **Fragment (position in template sequence)** | **Template strain (GenBank accession number)** | **Primer sequences** | **Expression vector (restriction sites used for cloning)** |
|---|---|---|---|---|
| crecFib-4/11 | I (nt1-1221^{a}) | KR5, FAdV-4 reference strain (HE608152) | FP1-KR5: 5′- AAA CCT GTA TTT TCA GGG CAT GCT CCG AGC CCC TA-3′ | pFAST BAC HTb (*Ehel*/*Stul*) |
| | | | RP1-KR5: 5′- AAG TTT GCA CGG ATC CGC TGG ATG GTT GAT AGT AT-3′ | |
| | II (nt1468-1713) | 13/14796, FAdV-11 field strain | FP2-13/14796: 5′-TCC AGC GGG TCC GTG CAA ACT TTC CAA CCC GTA TT-3′ | |
| | | | RP2-13/14796: 5´-GTG AGC TCG TCG ACG TAG GTT AGG GTT GTG TTA AT | |

| | | | | |
|---|---|---|---|---|
| ^{a}position refers to fiber-2 gene \| | | | | |

Correct insertion of the segments into the vector was confirmed by Sanger sequencing across the multiple cloning site (LGC Genomics, Berlin, Germany).

The recombinant protein was expressed in *Spodoptera frugiperda* Sf9 cells and purified via the polyhistidine tag on affinity chromatography columns as described previously (Schachner et *al.,* 2014), and their concentration determined by Bradford assay (Thermo Scientific, Vienna, Austria).

Sequences of crecFib construct
crecFib-4/11 (for FAdV-4 fiber 2 was used)
N-terminus:
   nt1-1221 from FAdV-4 reference strain (KR5) (HE608152) 
ATGCTCCGAGCCCCTAAAAGAAGACATTCCGAAAACGGGCAGCCCGAGTCCGAAGCGGGACCTTCCCCGGCTCCAAT
CAAGCGCGCGAAACGCATGGTGAGAGCATCCCAGCTTGACCTGGTTTATCCTTTCGATTACGTGGCCGACCCCGTCG
GAGGGCTCAACCCGCCTTTTTTGGGCGGCTCCGGACCCCTAGTGGACCAGGGCGGTCAGCTTACGCTCAACGTCACC
GATCCCATCATCATCAAGAACAGATCGGTGGACTTGGCCCACGATCCCAGTCTCGATGTCAACGCCCAAGGTCAACT
GGCGGTGGCCGTTGACCCCGAAGGGGCCCTGGACATCACCCCCGATGGACTGGACGTCAAGGTCGACGGAGTAACCG
TGATGGTCAACGATGACTGGGAACTGGCCGTAAAAGTCGACCCGTCCGGCGGATTGGATTCCACTGCGGGCGGACTG
GGGGTCAGCGTGGACGACACCTTGCTCGTGGATCAGGGAGAACTGGGCGTACACCTCAACCAACAAGGACCCATCAC
TGCCGATAGCAGTGGTATCGACCTCGAGATCAATCCTAACATGTTCACGGTCAACACCTCGACCGGAAGCGGAGTGC
TGGAACTCAACCTAAAAGCGCAGGGAGGCATCCAAGCCGGCAGTTCGGGAGTGGGCGTTTCCGTGGATGAAAGCCTA
GAGATTGTCAACAACACGCTGGAAGTGAAACCGGATCCCAGCGGACCGCTTACGGTCTCCGCCAATGGCCTAGGGCT
GAAGTACGACAGCAATACCCTGGCGGTGACCGCGGGCGCTTTGACCGTAGTAGGAGGGGGAAGCGTCTCCACACCCA
TCGCTACTTTTGTCTCGGGAAGTCCCAGCCTCAACACCTACAATGCCACGATCGTCAATTCCAGCTCGCACCCCTTC
TCTTGTGCCTACTACCTTCAACAGTGGAACGTACAAGGGCTCCTTTTTACCTCCCTCTACGTGAAACTGGACAGCAC
CACCATGGGGACTCGCCCTGGGGACAACAGCTCCGCCAATGCCAAATGGTTCACCTTTTGGGTGTCCGCCTATCTCC
AGCAATGCAACCCCTCCGGGATTCAAGCGGGAACGGTCAGCCCCTCCACCGCCGCCCTCGCGGACTTTGAACCCATG
GCCAATAGGAGCGTGTCCAGCCCATGGACGTACTCGGCCAATGCATACTATCAACCATCCAGCGGA
C-terminus:
   nt1468-1713 from FAdV-11 field strain (strain 13/14796) 
TCCGTGCAAACTTTCCAACCCGTATTGACAGGAGATTGGGATACCAACACCTACAACCCAGGAACCGTTCAAGTCTG
CATACTGCCTCAAACCGTTGTGGGAGGCCAGTCGACCTTTGTTAACATGACATGTTATAACTTCCGGTGTCAAAATC
CTGGAATATTCAAGGTTGCTGCTAGTAGCGGCACATTCACTATCGGACCCATTTTCTACTCCTGTCCAACCAACAAA
TTAACACAACCCTAA
Complete sequence:

ATGCTCCGAGCCCCTAAAAGAAGACATTCCGAAAACGGGCAGCCCGAGTCCGAAGCGGGACCTTCCCCGGCTCCAAT
CAAGCGCGCGAAACGCATGGTGAGAGCATCCCAGCTTGACCTGGTTTATCCTTTCGATTACGTGGCCGACCCCGTCG
GAGGGCTCAACCCGCCTTTTTTGGGCGGCTCCGGACCCCTAGTGGACCAGGGCGGTCAGCTTACGCTCAACGTCACC
GATCCCATCATCATCAAGAACAGATCGGTGGACTTGGCCCACGATCCCAGTCTCGATGTCAACGCCCAAGGTCAACT
GGCGGTGGCCGTTGACCCCGAAGGGGCCCTGGACATCACCCCCGATGGACTGGACGTCAAGGTCGACGGAGTAACCG
TGATGGTCAACGATGACTGGGAACTGGCCGTAAAAGTCGACCCGTCCGGCGGATTGGATTCCACTGCGGGCGGACTG
GGGGTCAGCGTGGACGACACCTTGCTCGTGGATCAGGGAGAACTGGGCGTACACCTCAACCAACAAGGACCCATCAC
TGCCGATAGCAGTGGTATCGACCTCGAGATCAATCCTAACATGTTCACGGTCAACACCTCGACCGGAAGCGGAGTGC
TGGAACTCAACCTAAAAGCGCAGGGAGGCATCCAAGCCGGCAGTTCGGGAGTGGGCGTTTCCGTGGATGAAAGCCTA
GAGATTGTCAACAACACGCTGGAAGTGAAACCGGATCCCAGCGGACCGCTTACGGTCTCCGCCAATGGCCTAGGGCT
GAAGTACGACAGCAATACCCTGGCGGTGACCGCGGGCGCTTTGACCGTAGTAGGAGGGGGAAGCGTCTCCACACCCA

TCGCTACTTTTGTCTCGGGAAGTCCCAGCCTCAACACCTACAATGCCACGATCGTCAATTCCAGCTCGCACCCCTTC
TCTTGTGCCTACTACCTTCAACAGTGGAACGTACAAGGGCTCCTTTTTACCTCCCTCTACGTGAAACTGGACAGCAC
CACCATGGGGACTCGCCCTGGGGACAACAGCTCCGCCAATGCCAAATGGTTCACCTTTTGGGTGTCCGCCTATCTCC
AGCAATGCAACCCCTCCGGGATTCAAGCGGGAACGGTCAGCCCCTCCACCGCCGCCCTCGCGGACTTTGAACCCATG
GCCAATAGGAGCGTGTCCAGCCCATGGACGTACTCGGCCAATGCATACTATCAACCATCCAGCGGATCCGTGCAAAC
TTTCCAACCCGTATTGACAGGAGATTGGGATACCAACACCTACAACCCAGGAACCGTTCAAGTCTGCATACTGCCTC
AAACCGTTGTGGGAGGCCAGTCGACCTTTGTTAACATGACATGTTATAACTTCCGGTGTCAAAATCCTGGAATATTC
AAGGTTGCTGCTAGTAGCGGCACATTCACTATCGGACCCATTTTCTACTCCTGTCCAACCAACAAATTAACACAACC
CTAA

### Virus preparation

Field isolates AG234 and 08-18926 (GenBank accession no. MK572849 and MK572871) were analyzed through Next-generation sequencing and virus-neutralization test (VNT) and thus identified as members of types FAdV-4 and -11, respectively (Schachner et *al.,* 2019), to be used as challenge strains during the protection studies. The strains were 3-fold plaque purified and propagated on primary chicken-embryo liver (CEL) cells (Schat and Sellers, 2008); viral titers were determined by endpoint titration (Reed and Muench, 1938).

### Clinical trial

One hundred twenty SPF chicks were hatched, individually tagged and divided into six groups (n = 20) under the following designation: vaccine-only, vaccine vs. FAdV-4, vaccine vs. FAdV-11, FAdV-4 challenge control, FAdV-11 challenge control, and negative control (Table 2).

**Table 2. Design of animal experiment.**

| **group** | **designation** | **vaccination** | **challenge strain** |
|---|---|---|---|
| I | vaccine-only | crecFib-4/11 | -^{a} |
| II | vaccine vs. FAdV-4 | crecFib-4/11 | AG234 (FAdV-4) |
| III | vaccine vs. FAdV-11 | crecFib-4/11 | 08-18926 (FAdV-11) |
| IV | challenge control FAdV-4 | adjuvant only | AG234 (FAdV-4) |
| V | challenge control FAdV-11 | adjuvant only | 08-18926 (FAdV-11) |
| VI | negative control | - | - |

| | | | |
|---|---|---|---|
| ^{a}not applicable | | | |

Each group was housed separately in isolator units (HM2500, Montair, The Netherlands). The vaccination consisted in a 0.5 ml injection in the *Musculus tibialis lateralis* containing 50 µg of crecFib-4/11 homogenized in an oil-based adjuvant and was carried out in day-old birds of vaccination groups, whereas birds of the challenge controls were injected with phosphate buffered saline (PBS) mixed with adjuvant, and negative control birds were administered sterile PBS only. A booster injection was administered in the same way at 7 days of life (6 dpv) in the three vaccinated groups, and birds of the challenge and negative controls received an injection of adjuvant mixed with PBS and PBS only, respectively, as described above. Blood was collected in weekly intervals from the second week of life up to challenge, then at each of the following sampling time points: 3, 5, 7 and 14 dpc. The birds were challenged at 22 days of life (15 dpb) using FAdV-4 strain AG234 (groups vaccine vs. FAdV-4, FAdV-4 challenge control) and FAdV-11 strain 08-18926 (groups vaccine vs. FAdV-11, FAdV-11 challenge control). In the time period after challenge, birds were monitored daily for clinical signs. Up to five birds per group were sacrificed and submitted to necropsy at 3, 5, 7 and 14 dpc, and individuals that were euthanized due to severe clinical affection were necropsied and sampled immediately.

### Clinical chemistry

Immediately before euthanasia, blood was collected from the jugular vein of each bird from the infected groups and negative control into heparin tubes (VACUETTE^{®}, Greiner Bio-One, Kremsmünster, Austria) to investigate the aspartate transaminase (AST) content in plasma as previously described (Matos et *al.,* 2016).

### Quantitative polymerase chain reaction (qPCR) from tissues of target organs

Tissue samples for quantification of viral load, including liver, spleen and bursa of Fabricius, were collected from birds of the infected groups and negative control, and stored at -20°C until processing. DNA extraction was performed with DNeasy Blood & Tissue Kit (Qiagen, Hilden, Germany) according to manufacturer's protocol, and the DNA was subsequently analyzed with an adapted qPCR assay based on the 52K gene (Günes et *al.,* 2012).

### Chimeric fiber enzyme-linked immunosorbent assay (ELISA) and virus neutralization test (VNT)

Sera collected during the experiments were tested on ELISA plates coated with crecFib-4/11 following the protocol described by Feichtner et *al.* (2018); the cut-off OD value was calculated from the sera of the negative control birds by computing the arithmetic mean plus three times the standard deviation. Samples from 21, 27 and 29-day old bird (respectively: pre-challenge, 5 dpc and 7 dpc) were also investigated for neutralizing antibodies (nAbs) as earlier described (Schachner et *al.,* 2014), with VNT against the strains that served as a template for fiber expression (vaccine strains) and the field isolates used as challenge strains during the protection studies (challenge strains).

### Statistical analyses

A preliminary analysis of the datasets was carried out using Shapiro-Wilk test together with a visual inspection of histograms and normal Q-Q plots in order to verify the normal distribution assumption. The mean values for organ-BW ratios, plasma AST and viral load in target organs of vaccinated groups were compared with the negative control and their respective challenge control group via unpaired Student's t-test. Mann-Whitney U test was used for the datasets that did not meet normality assumptions. In each case, *p* values ≤ 0.05 were considered statistically significant. Statistical analyses were performed with the software package SPSS Version 26 (IBM SPSS Statistics; IBM Corp., Armonk, New York, USA).

### RESULTS

### Clinical trial

In the FAdV-4 challenge control, clinical signs started at 2 dpc with mild depression in two birds, and progressed across the group with 13 affected birds at 3 dpc, three of which showing severe depression; at 4 dpc there were nine affected birds, and two of them had to be euthanized due to their inability to move and take feed; at 5 dpc, three birds were still showing milder clinical signs (Table 3). In the FAdV-11 challenge control, only one bird was affected with mild depression at 4 dpc. Among the vaccinated groups, only one individual challenged with FAdV-4 showed signs of depression at 3 dpc. No clinical signs were recorded in the vaccine-only and the negative control groups throughout the whole experiment. The recorded parameters for the birds euthanized at 4 dpc were included in the 5 dpc clusters for statistical analyses.

**Table 3. Daily clinical signs for each experimental group in the time period after challenge. The number of affected individuals is indicated in brackets. No clinical signs were recorded before 2 dpc and after 5 dpc.**

| **group** | **designation** | **clinical scores** | | | |
|---|---|---|---|---|---|
| | | **2 dpc** | **3 dpc** | **4 dpc** | **5 dpc** |
| I | vaccine-only | _a | - | - | - |
| II | vaccine vs. FAdV-4 | - | depression (n = 1) | - | - |
| III | vaccine vs. FAdV-11 | - | - | - | - |
| IV | challenge control FAdV-4 | mild depression (n = 2) | mild depression (n = 9) | mild depression (n = 5) | mild depression (n = 2) |
| | | | depression (n = 1) | depression (n = 2) | depression (n = 1) |
| | | | severe depression (n = 3) | severe depression, bird unable to stand or take feed (n = 2)* | |
| V | challenge control FAdV-11 | - | - | mild depression (n = 1) | - |
| VI | negative control | - | - | - | - |

| | | | | | |
|---|---|---|---|---|---|
| ^{a}**no** clinical signs observed * birds were euthanized | | | | | |

Mean liver-body weight (BW) ratio was significantly increased from 3 to 7 dpc for the FAdV-4 challenge control compared to the negative control, and from 3 to 5 dpc for the vaccine vs. FAdV-4 group, whereas the spleen-BW ratio remained affected up to 7 dpc in both groups (Fig.9A, 10B). The FAdV-4 challenge control also showed a significant decrease in the organ-BW ratio of bursa of Fabricius, compared to the negative control, at 3 and 5 dpc, and of the thymus-BW ratio at 5 and 7 dpc, differently from the respective vaccinated group (Fig.9C, 10D). The FAdV-11 challenge control showed a significant increase of the liver-BW ratio at 3 and 7 dpc, and of the spleen-BW ratio from 3 to 7 dpc, whereas the vaccine vs. FAdV-11 group only showed significant affection of the spleen-BW ratio at 5 dpc.

### Plasma AST

Plasma AST was significantly increased compared to the negative control from 3 to 7 dpc for the FAdV-4 challenge control, and from 3 to 5 dpc for the vaccine vs. FAdV-4 group, although the values of the vaccinated birds were significantly lower compared to the FAdV-4 challenge control at these time points (Fig.10). At 7 dpc, the FAdV-11 challenge control was significantly increased compared to the negative control, whereas the vaccine vs. FAdV-11 group was significantly increased at 14 dpc.

### Viral load in target organs

Within the FAdV-4 system, mean viral load of vaccinated birds was significantly lower than the challenge control in liver at 5 dpc, in spleen at 3 dpc, and in bursa of Fabricius at both time points (Fig.11). No significant differences were recorded in the analyzed organs between FAdV-11-infected groups at any time point. At 14 dpc it was possible to detect viral DNA exclusively in the spleen of birds from FAdV-4-infected groups.

### Antibody response: ELISA and VNT

The cut-off value for crecFib-4/11 ELISA was calculated at OD 0.12. At 14-day old (7 dpb), the mean OD of the totality of vaccinated birds (n = 60) was OD 0.48±0.48, with 81.7% (49/60) of the birds above the cut-off (Fig.12). The day before challenge (21-day old, 14 dpb), the mean OD value of vaccinated birds raised up to 2.43±0.87 with all the birds being above the cut-off. Adjuvant-injected birds (n = 40) had mean OD values of 0.07±0.03 and 0.05±0.01 at 14- and 21-day old respectively. Mean OD of the vaccine-only group suffered a decrease at 3 dpc (1.70±1.09) before continuously increasing throughout the experiment, reaching OD 3.46±0.01 at 36-day old (29 dpb). After challenge, mean OD of the vaccine vs. FAdV-4 group raised to 3.09±0.42 at 3 dpc and reached the highest end of measurable values at 5 dpc (3.32±0.28), remaining then consistent in its plateau until the end of the experiment. In comparison, a sharp rise in ELISA-measured antibodies was observed in FAdV-4 challenge control birds from 5 to 14 dpc (mean OD 0.25±0.15 to 2.57±0.44), although they remained lower than the vaccinated group. Vaccinated birds challenged with FAdV-11 also quickly reached the plateau after infection (mean OD 3.24±0.13 at 3 dpc to 3.39±0.13 at 14 dpc), whereas FAdV-11 challenge control showed low levels of antibody development (peak at 14 dpc with mean OD 0.28±0.13). Sera of negative control birds remained below the cut-off value throughout the whole experiment.

The day prior challenge, only four vaccinated birds displayed some degree of nAbs against FAdV-11, one of them reacting against both vaccine and challenge strain (titers: 3 and 8 log₂ respectively), one only against the vaccine strain (titers: 5 log₂), and two against the challenge strain (titers: 3 and 9 log₂) (Fig.13). In contrast, there was no neutralizing activity against any of the FAdV-4 strains, and the vaccine-only group saw no nAbs development at any of the tested time points. The rise in nAbs against FAdV-4 started after challenge in the infected birds, with the vaccinated group and the challenge control showing similar values against both the challenge strain (mean titer: 5.07±2.09 log₂ vs. 4.57±1.83 log₂ at 5 dpc, and 7.56±1.51 log₂ vs. 6.90±0.99 log₂ at 7 dpc) and the vaccine strain (2.53±2.56 log₂ vs. 3.64±2.37 log₂ at 5 dpc, and 6.67+1.87 log₂ vs. 6.10±0.99 log₂ at 7 dpc). Experimental groups infected with FAdV-11 showed a similar pattern against the challenge strain, although titers in the vaccinated birds were slightly lower than in the challenge control (mean: 3.79±2.78 log₂ vs. 5.36±2.68 log₂ at 5 dpc, and 5.90±1.10 log₂ vs. 7.00±1.63 log₂ at 7 dpc), and mean titers were generally lower against the vaccine strain (0.29±1.07 log₂ vs. 2.80±2.10 log₂ at 5 dpc, and 0.50±1.29 log₂ vs. 2.30±2.11 log₂ at 7 dpc). Interestingly, a small number of vaccinated birds that were infected with FAdV-4 developed nAbs against FAdV-11 after challenge, with three individuals displaying nAbs against both strains and one only against the vaccine strain between 5 and 7 dpc. Data on the number of individuals exhibiting nAbs for each group are shown in Fig.13.

### CONCLUSIONS

The vaccine with chimeric fiber protein crecFib-4/11 was able to prevent clinical signs upon FAdV-4 infection in all immunized birds except one, in contrast with the challenge control, where several birds were affected at various time points, including severe depression that led to the euthanasia of two individuals. Despite hepatomegaly being present in both FAdV-4-infected groups in the early phase of infection, the vaccinated group experienced a faster recovery within a week post challenge. This tendency was reflected by plasma AST analyses, another indicator of hepatic health, as well as the significant drop of viral load in the liver of vaccinated birds compared to FAdV-4 challenge control in the same time period. In contrast, splenomegaly was registered in both vaccinated and unvaccinated birds; however, when considering this parameter, the double role of the spleen as lymphoid organ and target of the infection must be taken into account, as the magnitude of the immune response may have played a role in influencing the splenic size of vaccinated birds. In fact, the viral load in the spleen was generally lower in the vaccinated group. Moreover, the vaccine prevented HHS-caused atrophy of other major lymphoid organs, such as thymus and bursa of Fabricius, possibly by limiting viral spreading, as supported by significantly lower viral load in the bursa of the vaccinated group compared to FAdV-4 challenge control.

IBH infection with FAdV-11 had overall a milder effect than HHS. Only one bird of the challenge control, among all the ones infected with FAdV-11, showed signs of mild depression. However, the detrimental effect of the virus was evident with significantly increased liver- and spleen-BW ratio in the challenge control group, occasionally paired with a pathological increase of AST, a damage that was consistently prevented by the crecFib-4/11 vaccine, with the only exception of observed splenomegaly in the vaccinated group at 5 dpc.

Clinical protection provided by crecFib-4/11 against both HHS and IBH was linked to the induction of high and uniform levels of systemic antibodies against the vaccination antigen measured with in-house fiber ELISA immediately before challenge, with all the vaccinated birds developing OD values above the calculated cut-off. The overall levels of both vaccinated/challenged groups reached the highest-end of measurable values right after challenge, and plateaued to the end of the experiment. The inclusion of a vaccine-only group allowed the monitoring of antibody development for the whole time period and showed that ELISA-measured antibodies of vaccinated/unchallenged birds overlapped with the values of the vaccinated/challenged groups, confirming that such efficient humoral response was not necessarily due to the booster effect with the live virus. In fact, antibodies directed against crecFib-4/11 never reached the highest measurable ODs in the challenge controls. However, a consistent lack of neutralizing antibodies (nAbs) was observed in the vaccinated groups before challenge, as a very small number of birds exhibited neutralization against FAdV-11, and none against FAdV-4. Neutralization against FAdV-11 also appeared in a few vaccinated birds after infection with FAdV-4, indicating a delayed development of vaccine nAbs or a possible booster effect of the virus. In contrast, challenge control birds consistently showed neutralizing activity against the respective infection virus after challenge, with all surviving individuals exhibiting nAbs a week after infection.

Taken together, subunit vaccination with crecFib-4/11 protected the chickens from clinical signs and severe outcome of HHS, while at the same time limiting pathological affection from both HHS and IBH. Therefore, chimeric fibers were again confirmed to represent an efficient protection strategy to provide broad coverage against FAdVs, not only on heterotypic level, as previously demonstrated in Example 1, but also across the species boundary.

### Non-patent references

Bertran K, et al (2021) A 10-Year retrospective study of inclusion body hepatitis in meat-type chickens in Spain (2011-2021). Viruses 13:2170
Chen L, et al (2018) Immunogenicity and protective efficacy of recombinant fiber-2 protein in protecting SPF chickens against fowl adenovirus 4. Vaccine 36:1203-1208
De Luca C, et al (2020) Fowl adenovirus (FAdV) fiber-based vaccine against inclusion body hepatitis (IBH) provides type-specific protection guided by humoral immunity and regulation of B and T cell response. Vet Res 51:143
El Bakkouri M, et al (2008) Structure of the C-terminal head domain of the fowl adenovirus type 1 short fibre. Virology 378:169-176
Feichtner F, et al (2018) Development of sensitive indirect enzyme-linked immunosorbent assays for specific detection of antibodies against fowl adenovirus serotypes 1 and 4 in chickens. Avian Pathol 47:73-82
Gomis S, et al (2006) Inclusion body hepatitis as a primary disease in broilers in Saskatchewan, Canada. Avian Dis 50:550-555
Grafl B, et al (2020) Successful reproduction of adenoviral gizzard erosion in 20-week-old SPF layer-type chickens and efficacious prophylaxis due to live vaccination with apathogenic fowl adenovirus serotype 1 strain (CELO). Vaccine 38:143-149
Günes A, et al (2012) Real-time PCR assay for universal detection and quantitation of all five species of fowl adenoviruses (FAdV-A to FAdV-E). J Virol Methods 183:147-153
Gupta A, et al (2017) Immunogenicity and protective efficacy of virus-like particles and recombinant fiber proteins in broilerbreeder vaccination against fowl adenovirus (FAdV)-8b. Vaccine 35:2716-2722
Gupta A, et al (2018) Inactivated and live bivalent fowl adenovirus (FAdV8b + FAdV11) breeder vaccines provide broadspectrum protection in chicks against inclusion body hepatitis (IBH). Vaccine 36:744-750
Harrach B, et al, Family Adenoviridae. In: King AMQ, Adams MJ, Carstens EB, Lefkowitz EJ (eds) Virus Taxonomy: Ninth Report of the International Committee on Taxonomy of Viruses, 9th ed. Elsevier Academic Press, San Diego
Henry Dunand CJ, et al (2016) Both neutralizing and non-neutralizing human H7N9 influenza vaccine-induced monoclonal antibodies confer protection. Cell Host Microbe 19:800-813
Hess M, et al (1995) The avian adenovirus penton: two fibres and one base. J Mol Biol 252:379-385
Hess M (2020) Aviadenovirus infections. In: Swayne DE, Boulianne M, Logue CM, McDougald LR, Nair V, Suarez DL (eds) Diseases of Poultry, 14th ed. Wiley-Blackwell, Hoboken
Hong JS, et al (1996) Domains required for assembly of adenovirus type 2 fiber trimers. J Virol 70:7071-7078
Hu Z, et al (2020) Hemagglutinin-specific non-neutralizing antibody is essential for protection provided by inactivated and viral-vectored H7N9 Avian Influenza vaccines in chickens. Front Vet Sci 6:482
Kim M, et al (2014) An inactivated oil-emulsion fowl adenovirus serotype 4 vaccine provides broad cross-protection against various serotypes of fowl adenovirus. Vaccine 32:3564-3568
Marek et al (2012) Two fiber genes of nearly equal lengths are a common and distinctive feature of fowl adenovirus C members. Vet. Microbiol. 156: 411-417)
Matos M, et al (2016) Selected clinical chemistry analytes correlate with the pathogenesis of inclusion body hepatitis experimentally induced by fowl aviadenoviruses. Avian Pathol 45:520-529
Mittal D, et al (2014) Characterization of fowl adenoviruses associated with hydropericardium syndrome and inclusion body hepatitis in broiler chickens. Virusdisease 25:114-119
Mo J (2021) Historical investigation of fowl adenovirus outbreaks in South Korea from 2007 to 2021: A comprehensive review. Viruses 13:2256
Norrby E (1969) The relationship between the soluble antigens and the virion of adenovirus type 3. IV. Immunological complexity of soluble components. Virology 37:565-576
Pallister JA, et al (1993) Serological relationships within the group E fowl adenoviruses. Intervirology 36:84-90
Popowich S, et al (2018) Broad spectrum protection of broiler chickens against inclusion body hepatitis by immunizing their broiler breeder parents with a bivalent live fowl adenovirus vaccine. Res Vet Sci 118:262-269
Reed LJ, Muench H (1938) A simple method of estimating fifty percent endpoints. Am J Hyg 27:493-497
Schachner A, et al (2014) Recombinant FAdV-4 fiber-2 protein protects chickens against hepatitis-hydropericardium syndrome (HHS). Vaccine 32:1086-1092
Schachner A, et al (2016) Detailed molecular analyses of the hexon loop-1 and fibers of fowl aviadenoviruses reveal new insights into the antigenic relationship and confirm that specific genotypes are involved in field outbreaks of inclusion body hepatitis. Vet Microbiol 186:13-20
Schachner A, et al (2018) Fowl adenovirus-induced diseases and strategies for their control - a review on the current global situation. Avian Pathol 47:111-126
Schachner A, et al (2019) Fowl adenovirus (FAdV) recombination with intertypic crossovers in genomes of FAdV-D and FAdV-E, displaying hybrid serological phenotypes. Viruses 11:1094
Schat K, et al (2008) Cell culture methods. In: Dufour-Zavala L, Swayne DE, Glisson JR, Pearson JE, Reed WM, Jackwood MW, Woolcock PR (eds) A laboratory manual for the isolation and identification of avian pathogens, 5th ed. OmniPress Inc, Madison
Schonewille E, et al (2010) Specific-pathogen-free chickens vaccinated with a live FAdV-4 vaccine are fully protected against a severe challenge even in the absence of neutralizing antibodies. Avian Dis 54:905-10
Shah MS, et al (2012) A subunit vaccine against hydropericardium syndrome using adenovirus penton capsid protein. Vaccine 30:7153-7156
Steer PA, et al (2011) Application of high-resolution melting curve analysis for typing of fowl adenovirus in field cases of inclusion body hepatitis. Aust Vet J 89:184-192
Steer-Cope PA, et al (2019) Vaccination with FAdV-8a induces protection against inclusion body hepatitis caused by homologous and heterologous strains. Avian Pathol 48:396-405
Tian KY, et al (2020) Protection of chickens against hepatitis-hydropericardium syndrome and Newcastle disease with a recombinant Newcastle disease virus vaccine expressing the fowl adenovirus serotype 4 fiber-2 protein. Vaccine 38:1989-1997
Venne D (2013) Field data of the changing clinical picture over time of inclusion body hepatitis in Canada with an emphasis on diagnosis, prevention and trials on supportive treatments. In: Proceedings of the 24th Annual Australian Poultry Science Symposium, Sydney, New South Wales, Australia, February 2013. The Poultry Research Foundation and the World's Poultry Science Association, pp 222-229
Vindahl Kringelum J, et al (2012) Reliable B cell epitope predictions: impacts of method development and improved benchmarking. PLoS Comput Biol, doi 10.1371/journal.pcbi.1002829
Wang J, et al (2018) Variant serotypes of fowl adenovirus isolated from commercial poultry between 2007 and 2017 in some regions of China. Avian Dis 62:171-176
Wang X, et al (2018b) Immune protection efficacy of FAdV-4 surface proteins fiber-1, fiber-2, hexon and penton base. Virus Res 245:1-6
Wang P, et al (2018) A novel monoclonal antibody efficiently blocks the infection of serotype 4 fowl adenovirus by targeting fiber-2. Vet Res 49:29; doi 10.1186/s13567-018-0525-y
Wang X, et al (2019) Penton-dodecahedron of fowl adenovirus serotype 4 as a vaccine candidate for the control of related diseases. Vaccine 37:839-47

## Claims

1. A fowl adenovirus (FAdV) subunit vaccine, comprising at least a chimeric **FAdV** fiber protein and an adjuvant.

2. The vaccine of claim 1, wherein the chimeric **FAdV** fiber protein comprises an N-terminal fiber protein fragment from a first **FAdV** serotype and a C-terminal fiber protein fragment from a second **FAdV** serotype.

3. The vaccine of claim 2, wherein the first **FAdV** serotype and the second **FAdV** serotype belong to different **FAdV** species.

4. The vaccine of claim 3, wherein the first **FAdV** serotype belongs to the **FAdV** species FAdV-C and the second **FAdV** serotype belongs to an **FAdV** species selected from FAdV-B, FAdV-D and FAdV-E, or vice versa; or wherein the first **FAdV** serotype belongs to the **FAdV** species FAdV-A and the second **FAdV** serotype belongs to an **FAdV** species selected from FAdV-B, FAdV-D and FAdV-E, or vice versa; or wherein the first **FAdV** serotype belongs to the **FAdV** species FAdV-C and the second **FAdV** serotype belongs to the **FAdV** species FAdV-A, or vice versa.

5. The vaccine of any one of claims 2 to 4, wherein the first **FAdV** serotype is FAdV-4 and the second **FAdV** serotype is selected from FAdV-2, FAdV-11, FAdV-8a and FAdV-8b, or vice versa; or wherein the first **FAdV** serotype is FAdV-1 and the second **FAdV** serotype is selected from FAdV-2, FAdV-11, FAdV-8a and FAdV-8b, or vice versa; or wherein the first **FAdV** serotype is FAdV-4 and the second **FAdV** serotype is FAdV-1, or vice versa.

6. The vaccine of any one of claims 2 to 4, wherein the first **FAdV** serotype is selected from FAdV-2, FAdV-11, FAdV-8a and FAdV-8b and the second **FAdV** serotype is selected from FAdV-2, FAdV-11, FAdV-8a and FAdV-8b, or vice versa, under the proviso that two different **FAdV** serotypes are selected.

7. The vaccine of any one of claims 1 to 6, wherein the N-terminal fiber protein fragment comprises a fiber shaft domain and an N-terminal part of a fiber knob domain, wherein the C-terminal fiber protein fragment comprises a C-terminal part of a fiber knob domain.

8. The vaccine of any one of claims 1 to 7, wherein the N-terminal fiber protein fragment comprises an amino-acid sequence at least 70%, preferably at least 80%, more preferably at least 90%, even more preferably at least 95% or even 97.5%, especially 99% or even completely identical to the sequence identified by any one of SEQ ID NOs: 1-4.

9. The vaccine of any one of claims 1 to 8, wherein the C-terminal fiber protein fragment comprises an amino-acid sequence at least 70%, preferably at least 80%, more preferably at least 90%, even more preferably at least 95% or even 97.5%, especially 99% or even completely identical to the sequence identified by any one of SEQ ID NOs: 5-8.

10. The vaccine of any one of claims 1 to 9, wherein the vaccine is cross-protective.

11. The vaccine of any one of claims 1 to 10, for use in immunizing birds, preferably poultry, especially chickens against an **FAdV** infection.

12. The vaccine of any one of claims 1 to 10, for use in ameliorating or preventing adenoviral gizzard erosion (AGE), inclusion body hepatitis (IBH) and/or hepatitis-hydropericardium syndrome (HHS) in birds, preferably in poultry, especially in chickens.

13. A method of vaccinating a bird against an **FAdV** infection, comprising the steps of:
- obtaining the vaccine of any one of claims 1 to 10, and
- administering the vaccine to the bird.

14. A method of producing an **FAdV** subunit vaccine, comprising the steps of:
- expressing a chimeric **FAdV** fiber protein in an expression system,
- purifying the fiber protein, and
- combining the fiber protein with an adjuvant to obtain the **FAdV** subunit vaccine;
preferably wherein the **FAdV** subunit vaccine is the vaccine defined in any one of claims 1 to 10.

15. The method of claim 14, wherein the expression system is selected from baculovirus expression systems, bacterial expression systems, and yeast expression systems.
